# EUROPEAN PATENT APPLICATION

(11) **EP 1 913 962 A1**
(43) Date of publication of application: **23.04.2008**
(21) Application number: 06022118.1
(22) Date of filing: 22.10.2006
(51) Int. Cl.: A61L 31/10, A61L 31/16

(54) **Expandable medical device for the treatment and prevention of cardiovascular diseases**

(71) Applicant: Perelson, Ophir, Beverly Hills CA 90211 (US)
(72) Inventor: Perelson, Ophir, Beverly Hills CA 90211 (US)
(74) Representative: Arth, Hans-Lothar

(57) **Abstract**

The invention relates to expandable medical devices such as stents, bioresorbable stents or catheter balloons with or without crimped stents comprising the pharmaceutically active agent gemcitabine as pure compound or incorporated into a drug release matrix optionally in combination with other drugs for the treatment of cardiovascular diseases such as arteriosclerosis, atherosclerosis, stenosis or restenosis and methods for the manufacture of these expandable medical devices.

## Description

The invention relates to expandable medical devices such as stents, bioresorbable stents or catheter balloons comprising the pharmaceutically active agent gemcitabine optionally in combination with other drugs for the treatment of cardiovascular diseases such as arteriosclerosis, atherosclerosis, stenosis or restenosis and methods for the manufacture of these expandable medical devices.

### BACKGROUND OF THE INVENTION

Coronary artery disease (CAD) is the most common form of cardiovascular disease and the leading cause of death in the developed world. Most recent statistics also show CAD as the leading cause of premature and permanent disability among workers in developed economies.

Treatment options for CAD vary according to the severity of the disease, the location of any blockages in the blood vessels and the overall health of the patients. Options include medical management, surgical intervention (CABG) or percutaneous coronary intervention (PCI) with balloon angioplasty (PTCA) and stenting.

Initial success of elective PTCA ranges between 96% and 99%. However, there are two major drawbacks to the use of PTCA. The first is acute closure of the target vessel during treatment and the second is restenosis.

The cause of restenosis is multifactorial and may include the development of scar tissue, vessel re-coil or vessel remodeling. Restenosis of the treated vessel requires repeat procedures in approximately 20-50% of patients.

An exact description of the term of restenosis cannot be found in the technical literature. The most frequently used morphologic definition of the restenosis is the reduction of the vessel diameter to less than 50 % of the normal after successful PTCA (percutaneous transluminal coronary angioplasty). This is an empirically determined value whose hemodynamic relevance and relation to clinical pathology lacks a stable scientific foundation. In practice, the clinical aggravation of a patient is often considered as a sign of a restenosis of the formerly treated vessel segment.

The shift to the use of stents was made on the basis of evidence of effectiveness in relation to restenosis following PTCA. However, in-stent restenosis remains an important adverse event following insertions of coronary artery stents. This is usually due to intimal smooth muscle cell hyperplasia, that is, the growth of cellular matrix in and around a stent and a reaction to tissue injury. The underlying cause of the intimal smooth muscle cell hyperplasia is vascular smooth muscle injury and disruption of the integrity of the endothelial barrier and the underlying extracellular matrix. The overall disease process can be termed a hyperproliferative vascular disease because of the etiology of the disease process.

Extensive research has been devoted to development of drug-eluting stents (DES) in order to fight in-stent restenosis of bare metal stents. The DES may have a polymer coating which facilitates gradual release of a drug that prevents re-stenosis into the local tissue. Current the most commonly used drugs for this purpose, paclitaxel and sirolimus (rapamycin), interrupt cell progression and inhibit cell proliferation and therefore reduce in-stent restenosis (ISR).

Although balloon dilatation and stents reduce the risk of a reoccurring vessel occlusion, they are until the present day not capable of completely preventing such restenosis. At present only two nameable embodiments of drug-eluting stents are known, the paclitaxel eluting stent of Boston Scientific Corp. and the rapamycin eluting stent from Cordis Corporation. There are also some other anti-restenosis drugs in clinical trials and early stages of clinical use, such as biolimus A9, everolimus, tacrolimus, pimecrolimus, and zotarolimus which belong to the "limus" compound family. However, these "limus" drugs are not without problems.

Latest research findings from clinical trials indicate that drug-eluting stents reduce the need for re-intervention after PCI to a greater extent than bare metal stents. Unfortunately, DES are not without complications. The more recent clinical trials demonstrate a restenosis rate of 5-10%.

At the last congress for cardiology in Barcelona, Spain in September 2006 first indications were presented that these drug eluting systems may generate late thromboses and may not be as safe as expected. Consequently the development of new drug eluting systems which are effective in the treatment of stenosis and restenosis and which do not lead to late time complications is desired.

These problems prompted the research into methods to decrease or eliminate restenosis. This included the development of coronary artery stents, which is a small, metal prosthesis placed within the artery at the time of angioplasty, to scaffold the vessel open. The technology was developed to address the two key issues faced during PTCA, acute closure and restenosis.

The search for more efficient medication or a combination of medications continues.

Objective of the present invention is to provide an expandable medical device which is effective in the treatment or prevention of arteriosclerosis, atherosclerosis, stenosis or restenosis.

This objective is solved by the technical teaching of the independent claims of the present invention. Further advantageous embodiments of the invention result from the dependent claims, the description, the figures, as well as the examples.

Surprisingly it was found that the active agent gemcitabine is highly active in the treatment of cardiovascular diseases and cardiovascular disorders as well as for the treatment of cardiovascular injuries caused, for instance, by balloon dilatation or stent implantation.

The active agent gemcitabine is marketed under the trademark Gemzar^{®} and was developed by Eli Lilly and Company. The active agent gemcitabine was initially developed as an antiviral agent and was protected by the European patent No. EP 0 122 707 B1 which expired March 6, 2004.

Gemcitabine is a nucleoside now used in chemotherapy. Gemcitabine has the IUPAC name 4-amino-1-[3,3-difluoro-4-hydroxy-5-(hydroxymethyl)tetrahydrofuran-2-yl]-1 H-pyrimidin-2-one and the following chemical structure:

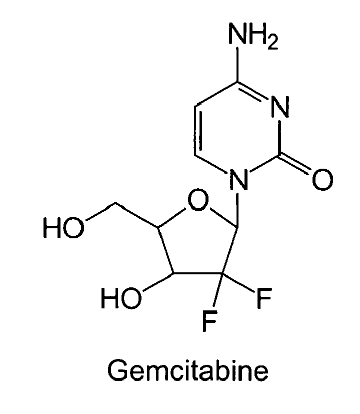

The chemical formula is: C₉H₁₁F₂N₃O₄

The molecular weight is: 263.198 g/mol

Gemcitabine is basic and can form salts with organic or inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

The term "gemcitabine" as used herein shall refer to the free base form of gemcitabine as well as to any kind of salt of gemcitabine. Normally the free base form or the hydrochloride salt or mixtures thereof are preferred for the inventive uses disclosed herein.

The term "gemcitabine compound" as used herein shall refer to gemcitabine itself any salt of gemcitabine and any gemcitabine derivative as well as any salt of any gemcitabine derivative. The term "gemcitabine derivative(s)" as used herein shall refer to gemcitabine itself any salt of gemcitabine and derivatives of gemcitabine such as amides, esters, ketals and acetals as exemplified by the general formula (I) to (VIII) as shown below. The following structures show possible derivatives of gemcitabine which fall under the term "gemcitabine" or "gemcitabine derivative(s)": 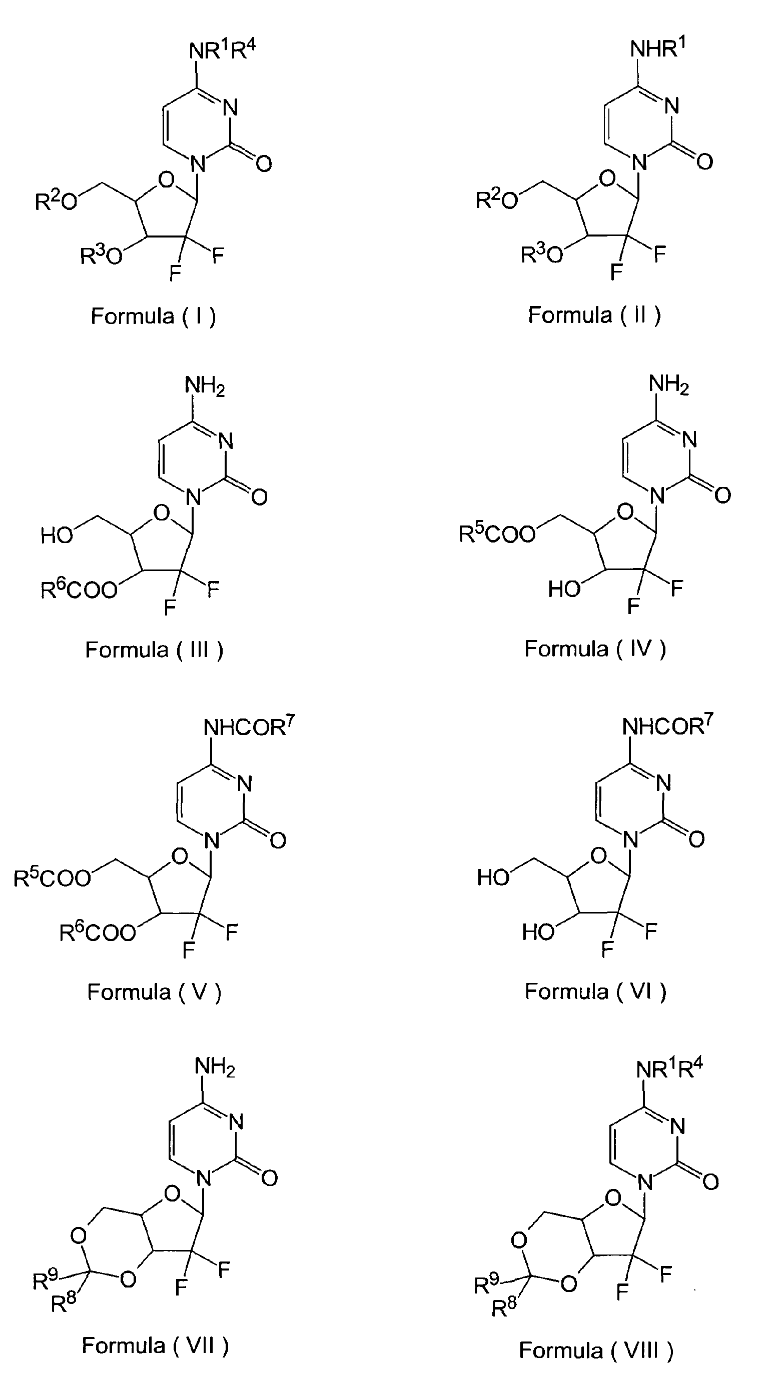 wherein R¹ to R⁴ represent hydrogen or nitrogen protecting groups, especially esters, amides, acetals or ketals.

Preferably R¹, R² and R³ are independently from each other selected from hydrogen and C₁ to C₃₀ saturated or monounsaturated or polyunsaturated acyl groups, perferably C₈ to C₂₆, and most preferably C₁₂ to C₂₄ saturated or monounsaturated or polyunsaturated acyl groups. Gemcitabine has three derivatisable functions, namely the 5'- and 3'-hydroxyl groups and the N⁴-amino group. Each group can selectively be transformed into an ester or amide derivative, but di-adducts (di-esters or ester-amides) and tri-adducts may be formed as well. In the case of the di- and tri-adducts the acyl substituent groups need not necessarily be the same.

However, the mono-acyl derivatives are preferred, i.e. with two of R¹, R² and R³ being hydrogen. It is especially preferred that the monosubstitution with the acyl group should be in the 3'-O and 5'-O positions of the sugar moiety, with 5'-O substitution being most preferred.

The double bond of the mono-unsaturated acyl groups may be in either the cis or the trans configuration. Furthermore, the use of esters or amides having their unsaturation in the [omega]-9 position are preferred. Especially preferred is the use of esters, ester-amides and amides derived from oleic acid (C18:1 [omega]-9, cis), elaidic acid (C18:1 [omega]-9, trans), eicosenoic acid(s) (C20:1 [omega]-9, cis) and (C20:1 [omega]-9, trans), and the amides and 5'-esters are currently the most preferred derivatives of gemcitabine. Esters, ester-amides and amides of gemcitabine derived from stearic acid (C18:0) and eicosanoic acid (C20:0) are also advantageously used.

Thus, one, two or three of the substituents R¹, R² and R³ are preferably carboxylic acid residues and most preferably fatty acid residues linked through an ester or amide bond respectively.

Preferred carboxylic acids are saturated, monounsaturated and polyunsatured acids as well as diacids and carbocycles or heterocycles containing carboxylic acids as well as carboxylic acids containing further functional groups such as hydroxy groups, amino groups or halogen groups.

Saturated fatty acids have commonly straight chains and even carbon number (n = 4 - 30). They have the general formula: CH₃(CH₂)ₙCOOH. Table 1 summarizes some saturated acids and their corresponding trivial names.

**Table 1: Most common saturated fatty acids**

| **Systematic name** | **Trivial name** | **Shorthand designation** |
|---|---|---|
| Butanoic acid | Butyric acid | 4:0 |
| Hexanoic ~ | Caproic ~ | 6:0 |
| Octanoic ~ | Caprylic ~ | 8:0 |
| Decanoic ~ | Capric ~ | 10:0 |
| Dodecanoic ~ | Lauric ~ | 12:0 |
| Tetradecanoic ~ | Myristic ~ | 14:0 |
| Hexadecanoic ~ | Palmitic ~ | 16:0 |
| Heptadecanoic ~ | Margaric ~ | 17:0 |
| Octadecanoic ~ | Stearic ~ | 18:0 |
| Eicosanoic ~ | Arachidic ~ | 20:0 |
| Docosanoic ~ | Behenic ~ | 22:0 |
| Tetracosanoic ~ | Lignoceric ~ | 24:0 |

Monoenoic fatty acids are monounsaturated normal fatty acids which are widespread in the living world where they occur mostly as their cis-isomers. They have the general structure CH₃(CH₂)ₓCH=CH(CH₂)_{y}COOH. They can have the unique double bond in a number of different positions, but the most common are of the n-9 series, as oleic acid from olive oil (cis-9-octadecenoic acid) and from most seed oils. Some important monoenoic acids are listed below:

**Table 2: Monoenoic fatty acids**

| **Systematic name** | **Trivial name** | **Shorthand designation** |
|---|---|---|
| cis-9-tetradecenoic acid | Myristoleic acid | 14:1 (n-5) |
| cis-9-hexadecenoic ~ | Palmitoleic ~ | 16:1 (n-7) |
| cis-6-octadecenoic ~ | Petroselinic ~ | 18:1 (n-12) |
| cis-9-octadecenoic ~ | Oleic ~ | 18:1 (n-9) |
| cis-11-octadecenoic ~ | Vaccenic ~ | 18:1(n-7) |
| cis-9-eicosenoic ~ | Gadoleic ~ | 20:1 (n-11) |
| cis-11-eicosenoic ~ | Gondoic ~ | 20:1(n-9) |
| cis-13-docosenoic ~ | Erucic ~ | 22:1(n-9) |
| cis-15-tetracosenoic ~ | Nervonic ~ | 24:1(n-9) |

Oleic acid is probably the most common fatty acid (60 - 70% in olive oil). Several positional isomers of oleic acid exist with the cis double bond in the (n-12) or (n-7) position but trans-isomers are known: Elaidic acid (t9-octadecenoic acid) and t-vaccenic acid (t11-octadecenoic acid) are found in the rumen and in lipids of ruminant animals. An unusual trans fatty acid, t3-hexadecenoic acid (trans-16:1 n-13), occurs in eukaryotic photosynthetic membranes from higher plants and green algae.

Polyenoic fatty acids are also called polyunsaturated fatty acids (PUFA). These fatty acids have 2 or more cis double bonds which are most frequently separated from each other by a single methylene group (methylene-interrupted polyenes). Linoleic acid is a typical member of this group. Some other polyunsaturated fatty acids undergo a migration of one of their double bonds which are not again methylene-interrupted and are known as conjugated fatty acids. Some unusual fatty acids do not have the regular structure with a methylene group between two double bonds but are polymethylene-interrupted polyenes. They are found in certain classes of plants, marine invertebrates and insects. Brominated long-chain fatty acids have been isolated from phospholipids of primitive marine animals such as sponges.

The most important polyenoic fatty acids can be grouped into 2 series with a common structural feature: CH₃(CH₂)_{×}CH=CH- with x=4 for the (n-6) series and with x=1 for the (n-3) series. Eicosapentaenoic acid is a common polyene of the (n-3) series having the double bonds in the 5, 8, 11, 14, and 17 positions. Table 3 summarizes the most common polyenoic fatty acids.

**Table 3: The commonest polyenoic fatty acids are listed below:**

| **Systematic name** | **Trivial name** | **Shorthand designation** |
|---|---|---|
| 9,12-octadecadienoic acid | Linoleic acid | 18:2(n-6) |
| 6,9,12-octadecatrienoic ~ | γ-Linolenic ~ | 18:3(n-6) |
| 8,11,14-eicosatrienoic ~ | Dihomo-γ-linolenic ~ | 20:3(n-6) |
| 5,8,11,14-eicosatetraenoic ~ | Arachidonic ~ | 20:4(n-6) |
| 7,10,13,16-docosatetraenoic ~ | - | 22:4(n-6) |
| 4,7,10,13,16-docosapentaenoic ~ | - | 22:5(n-6) |
| 9,12,15-octadecatrienoic ~ | α-Linolenic ~ | 18:3(n-3) |
| 6,9,12,15-octadecatetraenoic ~ | Stearidonic ~ | 18:4(n-3) |
| 8,11,14,17-eicosatetraenoic ~ | - | 20:4(n-3) |
| 5,8,11,14,17-eicosapentaenoic ~ | EPA | 20:5(n-3) |
| 7,10,13,16,19-docosapentaenoic ~ | DPA | 22:5(n-3) |
| 4,7,10,13,16,19-docosahexaenoic ~ | DHA | 22:6(n-3) |
| 5,8,11-eicosatrienoic ~ | Mead acid | 20:3(n-9) |

The most common polyene acids are octadecatrienoic acids (7 species are known). Eleostearic acid (9c11t13t) is found in tong oil and had an industrial importance, calendic acid (8t10t12c) is found in *Calendula officinalis* and catalpic acid (9c11t13c) is found in *Catalpa ovata.*

Recently, novel polyene fatty acids with different chain lengths and varying unsaturation were described: 16:5, 18:4, 20:5, 20:6, and unexpectedly 22:7. All these species have in common 4 conjugated all-cis double bonds as in 18:4 with their position in 6, 8, 10, and 12, the novel conjugated docosaheptadecanoic acid having its double bonds in 4, 7, 9, 11, 13, 16, and 19, was named stellaheptaenoic acid.

Among the unsaturated polymethylene-interrupted fatty acids found in the plant kingdom those with a cis-5 ethylenic bond are present in various sources. The three most frequent fatty acids with that structure are taxoleic acid (all-cis-5,9-18:2), pinolenic acid (all-cis-5,9,12-18:3) which is found in seeds of conifers, *Teucrium* and also in tall oil, and sciadonic acid (all-cis-5,11,14-20:3). These fatty acids are present in seed oil at levels from about 1% up to 25%. Similar species with 4 double bonds are also described.

Some isoprenoid fatty acids are known. In this group, the most interesting is retinoic acid which derives from retinol and has important functions in cell regulation.

Further suitable carboxylic acids are, for instance, 14-methyl pentadecanoic acid (isopalmitic acid), 13-methyl pentadecanoic acid, pristanic acid, phytanic acid, cyclopropane fatty acid such as lactobacillic acid (11,12-methyleneoctadecanoic acid) and 9,10-methylenehexa-decanoic acid, epoxy acids such as 9,10-epoxystearic and 9,10-epoxyoctadec-12-enoic (coronaric acid), carboxylic acids with further functional groups such as 2-hydroxy acids (or α-hydroxy acids) such as 2-hydroxytetracosanoic acid (cerebronic acid) and 2-hydroxy-15-tetracosenoic acid (hydroxynervonic acid) or 3-hydroxy acids (or β-hydroxy acids) such as ricinoleic acid (12-hydroxy-9-octadecenoic acid) and lesquerolic acid, the C20 homologue of ricinoleic acid (14-hydroxy-11-eicosenoic acid), heterocyclic fatty acids such as lipoic acid which is also known as thioctic acid or 1,2-dithiolane-3-pentanoic acid. After its absorption, this acid is reduced enzymatically by NADH or NADPH to dihydrolipoic acid (or 6,8-dithiane octanoic acid) in various tissues.

Acetylenic fatty acids, also known as ethynoic acids, include fatty acids which contain a triple bond and eventually one or two double bonds. Table 4 shows further examples of acetylenic fatty acids.

**Table 4: Acetylenic fatty acids**

| **Systematic name** | **Trivial name** |
|---|---|
| 6-octadecynoic acid | Tariric acid |
| t11-octadecen-9-ynoic ~ | Santalbic or Ximenynic ~ |
| 9-octadecynoic ~ | Stearolic ~ |
| 6-octadecen-9-ynoic ~ | 6,9-octadecenynoic ~ |
| t10-heptadecen-8-ynoic ~ | Pyrulic ~ |
| 9-octadecen-12-ynoic ~ | Crepenynic ~ |
| t7,t11-octadecadiene-9-ynoic ~ | Heisteric ~ |
| t8,t10-octadecadiene-12-ynoic ~ | - |
| 5,8,11,14-eicosatetraynoic ~ | ETYA |

Although the dicarboxylic acids do not occur in appreciable amounts as components of animal or vegetal lipids, they are in general important metabolic products of fatty acids since they originate from them by oxidation. They have the general type formula: HOOC-(CH₂)ₙ-COOH. Short-chain dicarboxylic acids are of great importance in the general metabolism and up to n=3 they cannot be considered as lipids since their water solubility is important. The simplest of these intermediates is oxalic acid (n=0), the others are malonic (n=1), succinic (n=2) and glutaric (n=3) acids. The other lipid members of the group found in natural products or from synthesis have a "n" value from 4 up to 21. Examples thereof are: adipic acid (n=4), pimelic acid (n=5), suberic acid (n=6), azelaic acid (n=7), sebacic acid (n=8), brassylic acid (n=11), and thapsic acid (n=14).

Examples of carboxylic acid derivatives of gemcitabine are described for example in US 2002/0042391 A1.

Furthermore the substituents R² and R³ may form together a six-membered acetal or ketal ring system as shown in general formula (VII) or (VIII). Therein R⁸ and R⁹ normally represent independently of each other hydrogen or a C₁- C₆ alkyl, C₃- C₇ cycloalkyl or C₆- C₁₃ arylalkyl group.

Furthermore gemcitabine derivatives of general formula (II) and (VI) are preferred wherein the substituent R¹ or COR⁷ represents an oligopeptide chain or a polypeptide chain such as poly-L-glutamic acid-gemcitabine.

Gemcitabine derivatives wherein one, two and three substituents of R¹ to R³ are different from hydrogen are disclosed in J. Org. Chem. 1999, 64 (22), 8319-8322 with the title "Selective Protection of 2',2'-Difluorodeoxycytidine (Gemcitabine)".

The preferred route of administration of gemcitabine (and gemcitabine derivatives) is orally and intravenously. The half-life of gemcitabine ranges between about 32 - 94 minutes when administered by short infusion or about 245 - 638 minutes when administered via long infusion.

Chemically gemcitabine is a nucleoside in which the hydrogens on the 2' carbon of deoxycytidine are replaced by fluorides. As with fluorouracil and other analogues of pyrimidines, the drug replaces one of the building blocks of nucleic acids, in this case cytidine, during DNA replication. The process arrests tumor growth, as new nucleosides cannot be attached to the "faulty" nucleoside, resulting in apoptosis (cellular "suicide").

Gemcitabine exhibits cell phase specificity, primarily affecting cells undergoing DNA synthesis (S-phase) and also blocking the progression of cells through the G1/S-phase boundary. Gemcitabine is metabolized intracellularly by nucleoside kinases to the active diphosphate (dFdCDP) and triphosphate (dFdCTP) nucleosides. The cytologic effect of gemcitabine is attributed to a combination of two actions of the diphosphate and the triphosphate nucleosides, which leads to inhibition of DNA synthesis. First, gemcitabine diphosphate inhibits ribonucleotide reductase, which is responsible for catalyzing the reactions that generate the deoxynucleoside triphosphates for DNA synthesis. Inhibition of this enzyme by the diphosphate nucleoside causes a reduction in the concentrations of deoxynucleotides, including dCTP. Second, gemcitabine triphosphate competes with dCTP for incorporation into DNA.

Gemcitabine is used in various carcinomas such as non-small cell lung cancer, pancreatic cancer, breast cancer and exhibits antitumor and immunosuppressive activity. It is being investigated for use in oesophageal cancer, and is used experimentally in lymphomas and various other tumor types.

Now it is disclosed for the first time that gemcitabine is useful for prophylaxis and treatment of cardiovascular diseases and disorders.

Cardiovascular diseases and cardiovascular disorders where gemcitabine can be applied successfully are for example adult congenital heart disease, aneurysm, stable angina, unstable angina, angina pectoris, angioneurotic edema, aortic valve stenosis, aortic aneurysm, arrhythmia, arrhythmogenic right ventricular dysplasia, arteriosclerosis, atherosclerosis, arteriovenous malformations, atrial fibrillation, Behcet syndrome, bradycardia, cardiac tamponade, cardiomegaly, congestive cardiomyopathy, hypertrophic cardiomyopathy, restrictive cardiomyopathy, cardiovascular disease prevention, carotid stenosis, cerebral hemorrhage, Churg-Strauss syndrome, diabetes, Ebstein's Anomaly, Eisenmenger complex, cholesterol embolism, bacterial endocarditis, fibromuscular dysplasia, congenital heart defects, heart diseases, congestive heart failure, heart valve diseases, heart attack, epidural hematoma, hematoma, subdural, Hippel-Lindau disease, hyperemia, hyperproliferative vascular disease, hypertension, pulmonary hypertension, hypertrophic growth, left ventricular hypertrophy, right ventricular hypertrophy, hypoplastic left heart syndrome, hypotension, intermittent claudication, ischemic heart disease, Klippel-Trenaunay-Weber syndrome, lateral medullary syndrome, long QT syndrome mitral valve prolapse, moyamoya disease, mucocutaneous lymph node syndrome, myocardial infarction, myocardial ischemia, myocarditis, pericarditis, peripheral vascular diseases, phlebitis, polyarteritis nodosa, pulmonary atresia, Raynaud disease, restenosis, Sneddon syndrome, stenosis, superior vena cava syndrome, syndrome X, tachycardia, Takayasu's arteritis, hereditary hemorrhagic telangiectasia, telangiectasis, temporal arteritis, tetralogy of fallot, thromboangiitis obliterans, thrombosis, thromboembolism, tricuspid atresia, varicose veins, vascular diseases, vasculitis, vasospasm, ventricular fibrillation, Williams syndrome, peripheral vascular disease, varicose veins and leg ulcers, deep vein thrombosis, Wolff-Parkinson-White syndrome.

Especially useful is gemcitabine for prophylaxis and treatment of arteriosclerosis, atherosclerosis, arteriovenous malformations, stenosis, in-stent stenosis, restenosis which means recurrent stenosis, vascular occlusion and hyperproliferative vascular disease.

Furthermore, gemcitabine is very useful for the treatment of vascular injuries which are caused or induced by mechanically mediated vascular injury, vascular catheterization, vascular scraping, percutaneous transluminal coronary angioplasty or stenting, vascular surgery and vascular laser treatment.

Especially preferred is the local treatment of the afore-mentioned diseases, disorders and injuries.

The present invention provides also a method of preventing or treating hyperproliferative vascular disease by local administration or local delivery of an antiproliferative effective amount of gemicitabine alone as a monotherapy or in combination with other complimentary medications. The administration can be achieved by a drug carrier vehicle such as a vascular stent or catheter balloon.

Thus one aspect of the present invention is directed to a method for preventing and treating cardiovascular diseases, cardiovascular disorders and cardiovascular injuries such as arteriosclerosis, atherosclerosis, arteriovenous malformations, stenosis, in-stent stenosis, restenosis, hyperproliferative vascular disease as well as vascular injuries caused or induced by mechanically mediated vascular injury, vascular catheterization, vascular scraping, percutaneous transluminal coronary angioplasty, vascular surgery and vascular laser treatment by administering to a patient in need a pharmaceutically effective amount of gemcitabine. Most preferably, said pharmaceutically effective amount of gemcitabine is administered locally for instance via a drug release system such as drug loaded particles or drug containing coatings.

Furthermore, the administration of a cytostatic amount of gemcitabine is preferred over the administration of a cytotoxic amount of gemcitabine.

The gemcitabine administration can be combined with a systemic treatment of cardiovascular diseases and disorders or cardiovascular injuries or can be combined with the local application of at least another anti-proliferative, anti-migrative, anti-inflammatory, anti-phlogistic, anti-restenosis, anti-angiogenic and/or anti-thrombotic active agent.

Examples for anti-proliferative, anti-migrative, anti-inflammatory, anti-phlogistic, anti-restenosis, anti-angiogenic and/or anti-thrombotic active agents are: sirolimus (rapamycin), everolimus, somatostatin, tacrolimus, roxithromycin, dunaimycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoposide, teniposide, nimustine, carmustine, lomustine, cyclophosphamide, C-type natriuretic peptide (CNP), 4-hydroxycyclophosphamide, estramustine, melphalan, ifosfamide, trofosfamide, chlorambucil, bendamustine, dacarbazine, busulfan, procarbazine, treosulfan, temozolomide, thiotepa, daunorubicin, doxorubicin, aclarubicin, epirubicin, mitoxantrone, idarubicin, bleomycin, mitomycin, dactinomycin, methotrexate, fludarabine, fludarabine-5'-dihydrogenphosphate, cladribine, mercaptopurine, thioguanine, cytarabine, fluorouracil, capecitabine, docetaxel, carboplatin, cisplatin, cryptophycine, anginex, oxaliplatin, amsacrine, irinotecan, topotecan, hydroxycarbamide, miltefosine, pentostatin, aldesleukin, tretinoin, asparaginase, pegaspargase, anastrozole, exemestane, letrozole, formestane, aminoglutethimide, adriamycin, azithromycin, spiramycin, cepharantin, smc proliferation inhibitor-2w, epothilone A and B, mitoxantrone, azathioprine, mycophenolatmofetil, c-myc-antisense, b-myc-antisense, betulinic acid, camptothecin, lapachol, β-lapachone, podophyllotoxin, betulin, podophyllic acid 2-ethylhydrazide, molgramostim (rhuGM-CSF), peginterferon α-2b, lenograstim (r-HuG-CSF), filgrastim, macrogol, anginex, Na-Uretic peptides, dacarbazine, basiliximab, daclizumab, selectin (cytokine antagonist), chryptophycines, CETP inhibitor, cadherines, cytokinin inhibitors, COX-2 inhibitor, AE-941 *(Neovastat^{®}) NFkB, angiopeptin, ciprofloxacin, camptothecin, fluroblastin, monoclonal antibodies, which inhibit the muscle cell proliferation, bFGF antagonists, probucol, prostaglandins, Ac-YVAD-CMK, 1,11-dimethoxycanthin-6-one, 1-hydroxy-11-methoxycanthin-6-one, scopoletin, colchicine, NO donors such as pentaerythritol tetranitrate and syndnoeimines, S-nitrosoderivatives, tamoxifen, staurosporine, β-estradiol, α-estradiol, estriol, estrone, ethinylestradiol, fosfestrol, medroxyprogesterone, estradiol cypionates, estradiol benzoates, tranilast, kamebakaurin and other terpenoids, which are applied in the therapy of cancer, verapamil, tyrosine kinase inhibitors (tyrphostines), cyclosporine A, paclitaxel and its derivatives such as 6-α-hydroxy-paclitaxel, baccatin, taxotere and others, synthetically produced as well as macrocyclic oligomers obtained from native sources of carbon suboxide (MCS) and its derivatives, mofebutazone, acemetacin, diclofenac, lonazolac, dapsone, o-carbamoylphenoxyacetic acid, lidocaine, ketoprofen, mefenamic acid, piroxicam, meloxicam, chloroquine phosphate, penicillamine, hydroxychloroquine, auranofin, sodium aurothiomalate, oxaceprol, celecoxib, β-sitosterin, ademetionine, myrtecaine, polidocanol, nonivamide, levomenthol, benzocaine, aescin, ellipticine, D-24851 (Calbiochem), colcemid, cytochalasin A-E, indanocine, nocodazole, S 100 protein, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor-1, plasminogen activator inhibitor-2, antisense oligonucleotides, VEGF inhibitors, IGF-1, active agents from the group of antibiotics such as cefadroxil, cefazolin, cefaclor, cefotaxim, tobramycin, gentamycin, penicillins such as dicloxacillin, oxacillin, sulfonamides, metronidazol, antithrombotics such as argatroban, aspirin, abciximab, synthetic antithrombin, bivalirudin, coumadin, enoxaparin, desulphated and N-reacetylated heparin (hemoparin^{®}), tissue plasminogen activator, GpIIb/IIIa platelet membrane receptor, factor Xₐ inhibitor antibody, heparin, hirudin, r-hirudin, PPACK, protamin, prourokinase, streptokinase, warfarin, urokinase, vasodilators such as dipyramidole, triazolopyrimidine (trapidil^{®}), nitroprussides, PDGF antagonists such as triazolopyrimidine and seramin, ACE inhibitors such as captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators such as p65 NF-kB or Bcl-xL antisense oligonucleotides, halofuginone, nifedipine, tocopherol, tranilast, molsidomine, tea polyphenols, epicatechin gallate, epigallocatechin gallate, Boswellic acids and its derivatives, leflunomide, anakinra, etanercept, sulfasalazine, etoposide, dicloxacillin, tetracycline, triamcinolone, mutamycin, procainamid, retinoic acid, quinidine, disopyrimide, flecainide, propafenone, sotalol, amidorone, natural and synthetically produced steroids such as bryophyllin A, inotodiol, maquiroside A, ghalakinoside, mansonine, strebloside, hydrocortisone, betamethasone, dexamethasone, non-steroidal substances (NSAIDS) such as fenoprofen, ibuprofen, indomethacin, naproxen, phenylbutazone and other antiviral agents such as acyclovir, ganciclovir and zidovudine, antimycotics such as clotrimazole, flucytosine, griseofulvin, ketoconazole, miconazole, nystatin, terbinafine, antiprozoal agents such as chloroquine, mefloquine, quinine, moreover natural terpenoids such as hippocaesculin, barringtogenol-C21-angelate, 14-dehydroagrostistachin, agroskerin, agrostistachin, 17-hydroxyagrostistachin, ovatodiolids, 4,7-oxycycloanisomelic acid, baccharinoids B1, B2, B3 and B7, tubeimoside, bruceanol A, B and C, bruceantinoside C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-α-senecioyloxychaparrin, taxamairin A and B, regenilol, triptolide, furthermore cymarin, apocymarin, aristolochic acid, anopterin, hydroxyanopterin, anemonin, protoanemonin, berberine, cheliburin chloride, cictoxin, sinococuline, bombrestatin A and B, cudraisoflavone A, curcumin, dihydronitidine, nitidine chloride, 12-β-hydroxypregnadiene-4,16-diene-3,20-dione, bilobol, ginkgol, ginkgolic acid, helenalin, indicine, indicine-N-oxide, lasiocarpine, inotodiol, glycoside 1a, podophyllotoxin, justicidin A and B, larreatin, malloterin, mallotochromanol, isobutyrylmallotochromanol, maquiroside A, marchantin A, maytansine, lycoridicin, margetine, pancratistatin, liriodenine, bisparthenolidine, oxoushinsunine, aristolactam-All, bisparthenolidine, periplocoside A, bisparthenolidine, periplocoside A, ghalakinoside, ursolic acid, deoxypsorospermin, psychorubin, ricin A, sanguinarine, manwu wheat acid, methylsorbifolin, sphatheliachromen, stizophyllin, mansonine, strebloside, akagerine, dihydrousambarensine, hydroxyusambarine, strychnopentamine, strychnophylline, usambarine, usambarensine, berberine, liriodenine, oxoushinsunine, daphnoretin, lariciresinol, methoxylariciresinol, syringaresinol, umbelliferon, afromoson, acetylvismione B, desacetylvismione A, vismione A and B and mixtures of these agents.

Especially preferred is the combination of gemcitabine especially gemcitabine hydrochloride in combination with one or more of cyclosporin A, tacrolimus, mycophenolate mofetil, daclizumab, rapamycin and/or one or more corticosteroid(s).

As such, gemicitabine alone or in any combination with for example paclitaxel and/or rapamycin or other medications is useful in preventing or treating intimal smooth muscle cell hyperplasia, restenosis, and vascular occlusion, particularly following either biologically or mechanically mediated vascular injury, such as during PCI. Mechanically mediated vascular injury includes, but is not limited to vascular injury caused by catheterization procedures or vascular scraping procedures such as percutaneous transluminal coronary angioplasty or stenting; vascular surgery; transplantation surgery; laser treatment; and other invasive procedures which disrupt the integrity of the vascular intima or endothelium.

The term "preventing" as used herein includes the prophylactic prevention of hyperproliferative vascular disease in a susceptible individual and treating includes arresting the development, and retarding the progression of hyperproliferative vascular disease.

The present invention is also directed to the use of gemcitabine for the manufacture of a pharmaceutical composition for prophylaxis and/or treatment of cardiovascular diseases and disorders and cardiovascular injuries.

The term "cardiovascular disease" as used herein shall also refer to hyperproliferative vascular diseases which may be caused by a mechanically mediated vascular injury.

Said pharmaceutical compositions may comprise at least one pharmaceutically acceptable carrier, excipient or diluent. Such pharmaceutically acceptable carrier, excipient or diluent can be selected from the group consisting of or comprising contrast agents, lactose, starch, sucrose, magnesium stearate, dicalcium phosphate, calcium sulfate, talc, mannitol, magnesium aluminum silicate, natural sugars, acacia, polyethylene glycol, waxes, guar gum, boric acid, sodium benzoate, sodium acetate, sodium chloride, acacia, gelatin, tragacanth, alginic acid, sodium alginate, ammonium calcium alginate, cellulose, methylcellulose, sodium carboxymethylcellulose, hydroxypropylmethylcellulose, polyvinylpyrrolidone.

The term "pharmaceutically acceptable carrier" as used herein shall also refer to an expandable medical device such as a stent, graft or dilatation balloon.

These pharmaceutical compositions can be administered orally, parenterally, intravascularly, intranasally, intrabronchially, transdermally, rectally, or via an expandable medical device impregnated or coated with gemcitabine or the gemcitabine-containing pharmaceutical composition.

Since local delivery of gemcitabine is preferred, an important aspect of the present invention relates to the use of gemcitabine as pharmaceutically active agent on an expandable medical device.

Examples of expandable medical devices are grafts, vascular grafts, stents, vascular stents, urological stents, biliary stents, bronchial stents, urinary stents, gastrointestinal stents, peripheral stents, catheter balloons, PTCA balloons, angiography ballons, and dilatation balloons.

These expandable medical devices can be coated with gemcitabine as a pure chemical substance or with gemcitabine incorporated into a polymeric matrix which may form a polymeric drug release system. Moreover gemcitabine could be blended with at least one pharmaceutically acceptable carrier and/or contrast agent. This mixture could then be used for the coating of the surface of an expandable medical device. These embodiments are described below in more detail.

Since the present studies exhibited that the mechanism of action of gemcitabine is favorable in controlling smooth muscle cell proliferation in the vascular wall, inhibiting intimal hyperplasia and reducing the incidence of in-stent restenosis the use of gemcitabine alone or in combination with at least one other anti-proliferative, anti-migrative, anti-inflammatory, anti-phlogistic, anti-restenosis, anti-angiogenic and/or anti-thrombotic active agent as disclosed above is quite favorable.

Consequently, a major aspect of the present invention is directed to a local release or delivery of gemcitabine alone or in combination with other drugs or in combination with other medications, wherein the local delivery or release is obtained by means of an expandable medical device such as metallic stents with a biostable polymer coating or bioabsorbable polymer coating or a fully degradable stent platform made from a metal, polymer or any other applicable material. Gemcitabine alone or a drug combination containing gemcitabine can also be directly applied on and directly released from the metallic surface of a stent without or with utilization of a base non-polymeric coating.

Thus, preferred expandable medical devices for release and delivery of gemcitabine are stents, grafts and balloons, especially vascular grafts, vascular stents, peripheral vascular stents, catheter balloons, PTCA balloons, angiography balloons and dilatation balloons.

The afore-mentioned balloons normally consist of a polymeric material such as polyamide, polyester, polyethylene and other commonly used balloon materials and the surface of said balloons can be coated or impregnated with gemcitabine or compound mixtures containing gemcitabine.

The coating methods applied for polymer and/or drug application are spray coating, dip coating, vapor deposition, plasma deposition, ion implantation, ultrasound-assisted, brush coating or filament coating.

The filament coating is described in detail in EP 1 518 517 A2. This coating method uses a spray nozzle for a continuous delivery of a paste containing the pharmaceutically active agent and this paste is selectively placed on the outer surface of the stent struts while the nozzle moves along the single filaments (stent struts). The method employs an automated controller to regulate the flow of a polymer or polymer-drug solution though the nozzle onto the filaments of a stent body, to achieve a uniform thickness coating on one side of the stent filaments and greater coating thickness on the outer surface of the stent body. Normally the drugrelease coating has a thickness of between 3-25 µm and is composed of 20 to 90 weight percent polymeric material.

If catheter balloons, PTCA balloons, angiography balloons or dilatation balloons shall be coated with gemcitabine, a liquid solution of gemcitabine in a solvent such as water, ethanol, acetone, isopropanol, tetrahydrofurane or the like is used for the coating process.

As non-polymeric matrix for the incorporation of gemcitabine contrast agents are preferred.

Useful contrast agents are selected from the group of contrast agents used for X-ray enhancement (X-ray contrast agents) or for magnetic resonance tomography (MR contrast agents).

Especially preferred are iodine containing contrast agents used for angiography or phlebography and still more preferred nonionic, iodinated contrast agents for use in computed tomography (CT).

Examples for iodine containing contrast agents are amidotrizoic acid, iotrolan, iodoxamic acid, ioglycamic acid, iopromide (Ultravist^{®}), iopamidol, iohexol, ioxilan, iomeprol, ioversol, iobitridol, iothalamate, Jod-Lipiodol^{®} (known under the trademarks Gastrografin^{®} and Gastrolux^{®}).

Other suitable contrast agents are meglumine, Telebrix^{®}, Omnipaque^{®}, and gadolinium containing contrast agents such as gadoliniumdiethylentriaminopentanoic acid, gadopentenoic acid (GaDPTA), gadodiamide, meglumin-gadoterate, gadoteridol.

Contrast agents are well pharmaceutically tolerable substances which have the further advantage to make the expandable medical device visible by imaging techniques such as X-ray or MR or CT.

Furthermore it is possible to use gemcitabine in combination with other drugs, which can be selected from the list as disclosed above, on the surface of the balloon in the form of a drug release coating and/or in the folds or crinkles or wings of the balloon and/or within the interior of the balloon.

One specific embodiment is a catheter balloon having small pores or openings for the release of a pharmaceutical composition containing gemcitabine. Such a pharmaceutical composition which can be released through the pores or openings in the balloon during dilatation are preferably liquid formulations of at least one contrast agent wherein gemcitabine is dissolved or dispersed or physiological sodium chloride solutions wherein gemcitabine is dissolved.

An embodiment of such a balloon is described in detail in US 2004/260239, US2004/260239, US2004/044308, US2003/225338, US2006/079837, US2004/260277, WO03/004087, or US2003/083653.

Another special kind of balloon is represented by a so-called fold-balloon. Such a balloon has folds or crinkles or wings wherein or whereunder preferably solid or semisolid compounds or mixtures can be deposited.

Such special kinds of balloons are disclosed in the European patent EP 0 519 063 B1 and the international patent applications WO 03/059430 A1 and WO 94/23787 A1.

The paste, liquid solution or gel containing gemcitabine can be deposited into or under the folds or crinkles or wings of the ballon by a dipping, spraying or injection method.

In order to secure the coating contianing gemcitabine to be washed of during insertion of the catheter (during PCl) an outer coating such as a barrier coating can be applied on the surface of the balloon, i.e. on the coating contianing gemcitabine. Such a barrier coating consists preferably of at least one rapidly biodegradable polymer.

Suitable biodegradable polymers are disclosed further below.

Gemcitabine as pure active agent or in combination with at least one pharmaceutically acceptable carrier, excipient or diluent or together with a drug release matrix comprising oligomeric or polymeric substances and/or contrast agents is present on the surface of the catheter balloon, PTCA balloon, angiography balloon or dilatation balloon in an amount ranging from 0.1 µg to 500 µg per mm² of the surface of the balloon, preferably between 0.5 µg to 100 µg per mm² balloon surface, more preferably in the range of 1.0 µg to 20.0 µg per mm² balloon surface, and most preferably between 3.0 µg to 10.0 µg per mm² outer surface of the balloon.

In the case a fold-balloon is used, this balloon should have preferably three to five folds or crinkles or wings, wherein in the folds or crinkles or wings or under the folds or crinkles or wings a paste, viscous liquid, dispersion, gel-like formulation or other substance is placed containing between 10 µg to 400 µg per fold or per crinkle or per wing, preferably between 50 µg to 200 µg per fold or per crinkle or per wing, and most preferably between 75 µg to 125 µg per fold or per crinkle or per wing of gemcitabine.

A balloon that was coated when unfolded is produced without any impact on the coating, for example by using balloon membranes with preformed folds and bends whose structure is not lost due to dilatation and which allow the balloon membrane to refold at least loosely when the pressure is discharged from the balloon without requiring an external force as primary cause. It is only after this prefolding that the preformed folds are compressed by external pressure or by a vacuum. Folds are in no way required to hold the at least one gemcitabine compound. In addition refolding can be achieved using minor mechanical force by very smooth materials, and the tools used may also be wetted by slippery biocompatible liquids in which the active ingredients do not or, at least, do not well dissolve.

In accordance with another variant of the invention, the balloons of readily folded balloon catheters are coated by dipping them into low-viscosity solution containing at least one gemcitabine compound. Solvent and gemcitabine compound penetrate into the extremely dense folds where they form a uniform coat that contains a reproducible dose and is not damaged by any subsequent step. The solution or, after the solvent has dried, the coat that adheres to the outer surface may be left there or may be removed in another step so that only the active agent portion that sits inside the folds of the balloon is retained.

After coating, when the balloon is folded, a stent can be pulled over the balloon catheter and firmly pressed onto it. The only step still required is sterilization, e.g. using ethylene oxide.

In the case a normal balloon is used without folds, crinkles or wings the balloon is dipped into a solution containing at least one gemcitabine compound or is sprayed with a solution containing at least one gemcitabine compound and optionally a further active agent or other pharmaceutically acceptable carriers and/or excipients.

As mentioned above, further anti-proliferative, anti-migrative, anti-inflammatory, anti-phlogistic, anti-restenosis, anti-angiogenic and/or anti-thrombotic active agents could be present together with gemcitabine. Examples for such agents are listed above. Said at least one other anti-proliferative, anti-migrative, anti-inflammatory, anti-phlogistic, anti-restenosis, anti-angiogenic and/or anti-thrombotic active agent is contained in a pharmaceutically active concentration of 0.001 - 20 mg per 100 mg of the pharmaceutical composition. In the case said further active agent is comprised in the same coating layer as gemcitabine or in an additional coating layer above or below the gemcitabine layer, said at least one other anti-proliferative, anti-migrative, anti-inflammatory, anti-phlogistic, anti-restenosis, anti-angiogenic and/or anti-thrombotic active agent is contained in a pharmaceutically active concentration of 0.001 - 20 mg per cm² of the balloon surface, more preferred in the range of 0.005 - 15 mg and especially preferred 0.01 - 10 mg per cm² of the surface of the catheter balloon, PTCA balloon, angiography ballon or dilatation balloon.

In the case a contrast agent is used for the uptake of gemcitabine, this at least one kind of contrast agent is present in an 10-fold to 100-fold larger amount than gemcitabine or said at least one kind of contrast agent is present in the range between 0.01 mg / cm² to 3 mg / cm² of the balloon-surface, further preferred 0.20 mg to 1 mg / cm² and especially preferred 0.2 mg / cm² to 0.5 mg / cm² of the surface of the catheter balloon, PTCA balloon, angiography ballon or dilatation balloon.

If a polymeric or oligomeric carrier is used as a drug release coating or as a polymeric carrier on the balloon surface, the oligomeric and/or polymeric substances are deposited on the balloon surface in an amount ranging from 0.01 mg / cm² to 3 mg / cm² of the balloon-surface and per layer, further preferred between 0.20 mg to 1 mg / cm² and especially preferred 0.2 mg / layer and cm² to 0.5 mg / layer and cm² of the surface of the catheter balloon, PTCA balloon, angiography ballon or dilatation balloon.

Especially preferred embodiments of the present invention are expandable medical devices in form of tubular members, grafts or stents coated with gemcitabine, at least one gemcitabine compound or at least one gemcitabine derivative optionally together with a pharmaceutically acceptable carrier, excipient or diluent or optionally together with at least one contrast agent and/or one kind of oligomer or poymer.

Such stents or can be vascular grafts, vascular stents, urological stents, biliary stents, bronchial stents, urinary stents, gastrointestinal stents, peripheral stents, and are most preferably vascular stents.

Such stents can be manufactured of any material and material mixtures including biostable as well as biodegradable materials. The stent is in general uncoated and/or not or only conditionally hemocompatible. Particularly, the bare stent which is provided does not have a coating made of organic material.

Normally these stents are formed of metals and metal alloys such as for example medical stainless steel, titanium, chromium, vanadium, tungsten, molybdenum, gold and nitinol. Furthermore composite or sintered materials may be used as stent material. The stent can also be formed of biodegradable material such as magnesium alloys, zinc alloys or calcium alloys or of biodegradable organic material such as collagen, chitin, chitosan, heparin, heparan sulphates and the like.

A biodegradable magnesium stent is for instance disclosed in EP 1 419 793 B1 and DE 102 07 161 A1. A biodegradable zinc stent is disclosed in EP 0 966 979 A2 and biodegradable metal stents with a biodegradable coating is decribed in DE 102 37 572 A1. Other suitable metals are disclosed in DE 198 56 983 A1.

Before the gemcitabine or the at least one gemcitabine compound is applied on the stent surface, the normally not or only slighlt hemocompatible stent surface can be coated with a hemocompatible layer comprising heparin, chitosan, chitosan derivatives or heparin derivatives such as desulphated and reacylated heparin or heparan sulphate. Suitable compounds wich can be used for the generation of the hemocompatible base coat are disclosed in detail in the European patent No. EP 1 501 565 A1.

The basic embodiment of a coated stent or graft is a stent or graft which is coated with the active agent gemcitabine. The coating methods such as spray coating, dip coating, vapor deposition, plasma deposition, ion implantation, brush coating or filament coating are described above. Normally, spray coating is preferred.

For this purpose, a solution of the at least one gemcitabine compound is prepared in an organic solvent such as ethanol, acetone, isopropanol, tetrahydrofurane, chloroform, DMF, ether, ethylacetate, methylene chloride, toluene and the like and the solution is sprayed onto the stent or the stent is dipped therein. A coated stent will be obtained without any drug release coating or polymeric carrier or matrix for controlled drug release.

In other embodiments the gemcitabine compound is incorporated into a matrix which can be a non-polymeric matrix, a oligomeric matrix or a polymeric matrix. A non-polymeric matrix could be formed by the use of contrast agents as mentioned above. Contrast agents have further advantages such as they as physiologically well tolerable, they are already used during stent placement and PTCA and they make the stent visible by imaging technologies as outlined above.

In the case a contrast agent is used for the uptake of gemcitabine compound, this at least one kind of contrast agent is present in an 10-fold to 100-fold larger amount than the gemcitabine compound or said at least one kind of contrast agent is present in the range between 0.01 mg / cm² to 3 mg / cm² of the stent surface, further preferred 0.20 mg to 1 mg / cm² and especially preferred 0.2 mg / cm² to 0.5 mg / cm² of the surface of the stent.

Therefore a stent optionally coated with a hemocompatible base coat and a non-polymeric matrix comprising the at least one gemcitabine compound and at least one kind of contrast agents is a very advantageous embodiment. In order to prevent the contrast agent matrix system from being washed off too quickly during balloon insertion and dilation, said non-polymeric coating can be covered by a more stable and preferably biodegradable layer made of polymeric compounds. Such a barrier layer can be used to control the release of the gemcitabine compound and also the other active agents which may optionally be present.

Further very preferred embodiments of the present invention are grafts or stents coated with a polymeric or other synthetic carrier system for controlled release of the at least one gemcitabine compound. These stents may comprise a hemocompatible base coat as mentioned before.

The polymeric or oligomeric carrier is used for the incorporation of the at least one gemcitabine compound and optionally further active agents such as anti-proliferative, anti-migrative, anti-inflammatory, anti-phlogistic, anti-restenosis, anti-angiogenic and/or anti-thrombotic active agents as exemplified above.

The polymers or oligomers used for generating the drug carrier layer on the stent surface can roughly be divided into more biostable polymers and more biodegradable (or bioresorbable) polymers.

More biodegradable and less biostable substances for the biodegradable layer(s) are for instance polyvalerolactones, poly-ε-decalactones, polylactonic acid, polyglycolic acid, polylactides, polyglycolides, copolymers of the polylactides and polyglycolides, poly-ε-caprolactone, polyhydroxybutanoic acid, polyhydroxybutyrates, polyhydroxyvalerates, polyhydroxybutyrate-co-valerates, poly(1,4-dioxane-2,3-diones), poly(1,3-dioxane-2-one), poly-para-dioxanones, polyanhydrides as polymaleic anhydrides, polyhydroxymethacrylates, fibrin, polycyanoacrylates, polycaprolactonedimethylacrylates, poly-β-maleic acid, polycaprolactonebutylacrylates, multiblock polymers as e.g. from oligocaprolactonedioles and oligodioxanonedioles, polyetherester multiblock polymers as e.g. PEG and poly(butyleneterephtalates), polypivotolactones, polyglycolic acid trimethyl-carbonates, polycaprolactone-glycolides, poly(γ-ethylglutamate), poly(DTH-iminocarbonate), poly(DTE-co-DT-carbonate), poly(bisphenol-A-iminocarbonate), polyorthoesters, polyglycolic acid trimethyl-carbonates, polytrimethylcarbonates, polyiminocarbonates, poly(N-vinyl)-pyrrolidone, polyvinylalcohols, polyesteramides, glycolated polyesters, polyphosphoesters, polyphosphazenes, poly[p-carboxyphenoxy)propane], polyhydroxypentane acid, polyanhydrides, polyethyleneoxide-propyleneoxide, soft polyurethanes, polyurethanes with amino acid residues in the backbone, polyetheresters as polyethyleneoxide, polyalkeneoxalates, polyorthoesters as well as their copolymers, lipids, carrageenans, fibrinogen, starch, collagen, protein based polymers, polyamino acids, synthetic polyamino acids, zein, modified zein, polyhydroxyalkanoates, pectic acid, actinic acid, modified and non modified fibrin and casein, carboxymethylsulphate, albumin, moreover hyaluronic acid, chitosan and its derivatives, heparansulphates and its derivatives, heparin, chondroitinsulphate, dextran, β-cyclodextrins, copolymers with PEG and polypropyleneglycol, gum arabicum, guar, gelatine, collagen, collagen-N-hydroxysuccinimide, lipids, phospholipids, modifications and copolymers and/or mixtures of afore mentioned substances are used.

Examples for more biostable and less biodegradable substances for the biostable layer(s) are polyacrylic acid and polyacrylates as polymethylmethacrylate, polybutylmethacrylate, polyacrylamide, polyacrylonitriles, polyamides, polyetheramides, polyethylenamine, polyimides, polycarbonates, polycarbourethanes, polyvinylketones, polyvinylhalogenides, polyvinylidenhalogenides, polyvinylethers, polyisobutylenes, polyvinylaromates, polyvinylesters, polyvinylpyrollidones, polyoxymethylenes, polytetramethyleneoxide, polyethylene, polypropylene, polytetrafluoroethylene, polyurethanes, polyetherurethanes, silicone-polyetherurethanes, silicone-polyurethanes, silicone-polycarbonate-urethanes, polyolefine elastomeres, polyisobutylenes, EPDM gums, fluorosilicones, carboxymethylchitosans, polyaryletheretherketones, polyetheretherketones, polyethylenterephthalate, polyvalerates, carboxymethylcellulose, cellulose, rayon, rayontriacetates, cellulosenitrates, celluloseacetates, hydroxyethylcellulose, cellulosebutyrates, celluloseacetatebutyrates, ethylvinylacetate copolymers, polysulphones, epoxy resins, ABS resins, EPDM gums, silicones as polysiloxanes, polydimethylsiloxanes, polyvinylhalogenes and copolymers, celluloseethers, cellulosetriacetates, chitosans and copolymers and/or mixtures of these substances are used.

Preferred substances are polyethersulfone, substituted polyethersulfone, polyphenylsulfone, substituted polyphenylsulfone, polysulfone block copolymers, perfluorinated polysulfone block copolymers, semifluorinated polysulfone block copolymers, substituted polysulfone block copolymers and/or mixtures of the aforementioned polymers.

Furthermore, polysulfones and especially thermoplastic polysulfones are preferred as polymers especially as more biostable polymers. Thermoplastic polysulfones can be deformed plastically (plastic) under the influence of heat (thermo). Generally, thermoplastic polysulfones consist of linear or less branched molecule chains. When heated, they can be extended by stretching. When further heated, they can be smelted completely and be rebuilt. In particular, it is preferred if these thermoplastic polysulfones have hydrophilic as well as hydrophobic properties. Such thermoplastic polysulfones having these ambivalent properties can be synthesized according to the above described methods via polymer analogous reactions, block copolymerizations or polymerization of hydrophilic with hydrophobic monomers. The thermoplastic polymers obtained in that way, or, respectively, the medical products coated therewith, distinguish by multiple sterilizability, resistance against hot steam and hydrolysis, high stability of dimension, resistance against aggressive chemicals as well as good thermal aging stability.

A preferred thermoplastic polysulfone is synthesized from bisphenol A and 4,4'-dichlorophenylsulfone via polycondensation reactions (see following formula (II)).

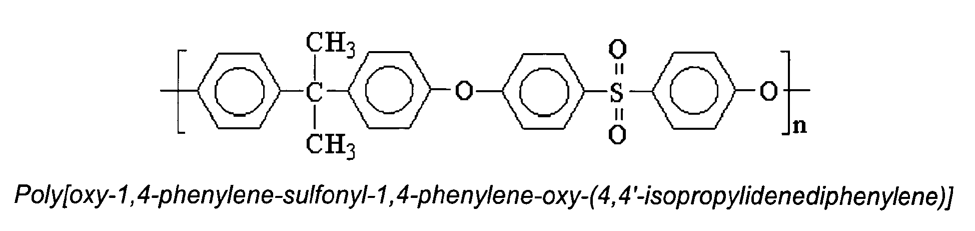

The polysulfones which are applicable for the coating according to the invention have the following general structure according to formula (1): 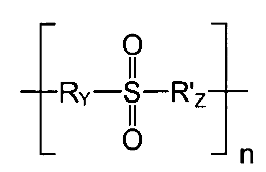 wherein
n represents the grade of polymerization, which is in the range from n = 10 to n = 10,000, preferably in the range from n = 20 to n = 3,000, further preferably in the range from n = 40 to n = 1,000, further preferably in the range from n = 60 to n = 500, further preferably in the range from n = 80 to n = 250 and particularly preferably in the range from n = 100 to n = 200.

Further, it is preferred if n is in such a range that a weight average of the polymer of 60,000 - 120,000 g/mol, preferably 70,000 to 99,000 g/mol, further preferably 80,000 - 97,000 g/mol, still more preferably 84,000 - 95,000 g/mol, and particularly preferably 86,000 - 93,000 g/mol results.

Moreover, it is preferred if n is in such a range that the number average of the polymer in a range from 20,000 - 70,000 g/mol, preferably from 30,000 - 65,000 g/mol, further preferably 32,000 - 60,000, still more preferred 35,000 - 59,000, and particularly preferably from 45,000 - 58,000 g/mol results.

The at least one gemcitabine compound can be used alone or in combination with gemcitabine, gemcitabine derivatives or another gemcitabine compound and/or in combination with other drugs such as paclitaxel, Rapamycin, cyclosporin A, tacrolimus, mycophenolate mofetil, daclizumab, rapamycin and/or one or more corticosteroid(s).

Said at least one further active agent can be present in the same layer as the gemcitabine compound or in a different layer which can be located under or on top of the gemcitabine compound containing layer.

As mentioned above, further anti-proliferative, anti-migrative, anti-inflammatory, anti-phlogistic, anti-restenosis, anti-angiogenic and/or anti-thrombotic active agents could be present together with gemcitabine compound. This further active agent is contained in a pharmaceutically active concentration of 0.001 - 20 mg per cm² of the stent surface, more preferred in the range of 0.005 - 15 mg and especially preferred 0.01 - 10 mg per cm² of the surface of the stent.

If a polymeric or oligomeric carrier is used as a drug release coating or as a polymeric carrier on the balloon surface, the oligomeric and/or polymeric substances are deposited on the stent surface in an amount ranging from 0.01 mg / cm² to 3 mg / cm² of the stent surface and per layer, further preferred between 0.20 mg to 1 mg / cm² and especially preferred 0.2 mg / layer and cm² to 0.5 mg / layer and cm² of the surface of the stent.

Normally the at least one gemcitabine compound is bound adhesively to the stent or the matrix. In a still further preferred embodiment the gemcitabine compound is covalently bound to the stent or to the polymeric carrier. The covalent linkage is achieved preferably through an ester bond via substituent R² or R³ or through an amide bond via substituent R¹.

The preferred embodiment of the stents according to the invention shows a coating, which consists of at least two layers. Thereby named as a second layer is that layer, which is deposited on the first layer. According to the two-layer design the first layer consists of the hemocompatible layer, which is substantially completely covered by a second layer, which consists of the at least one gemcitabine compound, that is covalently and/or adhesively bound to or in the second layer. Preferably a more biodegradable polymer is used for the second layer.

The gemcitabine compound containing layer is dissolved slowly, so that the active agent is released according to the velocity of the solution process. The first hemocompatible layer guarantees the necessary blood compatibility of the stent as the active agent is removed. By the release of the active agent the adhesion of cells is strongly reduced only for a certain period of time and an aimed controlled adhesion is enabled, where the external layer has been already widely degradated. Finally the hemocompatible layer remains an athrombogeneous surface and masks the foreign surface in such a way, that no life-threatening reaction can occur. The release of the gemcitabine compound spans a period from about 1 to 12 months, preferably 1 to 3 months after implantation.

If desired, a third coating (the outer coating) can be generated on the second coating in order to further control the release of the at least one gemcitabine compound and optionally the further active agent. Such an outer barrier layer preferably consists of biodegradable oligomers and/or polymers.

In the case a biostable or more biostable polymeric carrier for incorporation of the at least one gemcitabine compound and also optionally the further active agent is used, it is not necessary to have a hemocompatible base coat directly on the surface of the bare stent. However, an outer barrier layer could still be used for control of drug release. Said outer layer may contain a further active agent and can be manufactured of biostable or biodegradable polymers or oligomers or mixtures of biostable and biodegradable materials.

Suchlike stents can be generated by a method for coating expandable medical devices comprising the steps:
a) Providing an expandable medical device such as a stent, and
b) depositing gemcitabine or the gemcitabine compound as pure substance or in combination with at least one pharmaceutically acceptable carrier or with a polymeric carrier on the surface of the expandable medical device or in cavities in the surface of the expandable medical device or into folds or crinkles or wings of the expandable medical device.

If a hemocompatible coating is desired the method will change slightly and comprise the steps:
a) Providing an expandable medical device, and
a') applying a layer of a hemocompatible material on the surface of the expandable medical device, and
b) depositing gemcitabine as pure substance or in combination with at least one pharmaceutically acceptable carrier or with a polymeric carrier on the hemocompatible surface of the expandable medical device.

If an outer layer as a second or third layer is desired, said preferable outer barrier layer will be generated in a third step:
c) depositing at least one further layer of at least one biodegradable polymer on the gemcitabine layer or on the gemcitabine-containing layer.

Within these methods dipping and spraying methods are preferred.

In multi layer systems, the layer which has been newly deposited substantially covers the subjacent layer. "Substantially" means by 50 - 100%, preferably 70 - 100%, further preferred 80 - 100%, further preferably 90 - 100% and especially preferred up to over 96% and in particular further preferably up to over 98%.

The stents according to the invention solve both the problem of acute thrombosis and the problem of neointima hyperplasia after stent implantation. In addition the inventive stents are especially well suited, because of their coating for the continuous release of the at least one gemcitabine compound and optionally the one or more anti-proliferative, anti-migrative, anti-inflammatory, anti-phlogistic, anti-restenosis, anti-angiogenic and/or anti-thrombotic active agents. Due to this capability of aimed continuous active agent release in a required amount the inventively coated stents prevent the danger of restenosis almost completely.

### Examples

The following examples provide exemplary procedures for the coating of stents with the active agent gemcitabine. Stents directly coated with a pure active agent are described in Examples 1 and 2. Stents provided with a biodegradable and/or hemocompatible base coat are described in Examples 3 and 4. Stents directly coated with the active agent gemcitabine in a biodegradable matrix are described in Example 5. Stents prepared having a biodegradable and/or hemocompatible base coat coated with the active agent gemcitabine in a biodegradable matrix are described in Example 6. Stents prepared having a biodegradable and/or hemocompatible base coat, the active agent gemcitabine in a biodegradable matrix and a biodegradable top coat are described in Example 7. Examples 8, 9 and 10 describe the use of an alternative coating and matrix, polyethersulfone, with gemcitabine. Examples 11 and 12 describe the use of further active agents in addition to gemcitabine. Example 13 provides a protocol for the in vivo evaluation of gemcitabine. Examples 14 and 15 describe the coating of a balloon with gemcitabine and the coating of a catheter balloon with gemcitabine and a contrast agent.

The spray coating method as described in Examples 4 and 5 can also be substituted by the dip coating method and the dip coating method as described in Example 3 can also be substituted by the spray coating method.

### Example 1. Spray coating of bare metal stent with pure active agent

Preparation of spray solution: The spray solution is prepared by dissolving 44 mg gemcitabine in 6g chloroform.
Preparation of stents: Not expanded stents of medicinal stainless steel LVM 316, cobalt chromium alloy or other metallic compositon are degreased in the ultrasonic bath for 15 minutes with acetone and ethanol and dried at 100°C in the drying closet. Spraying method: The pre-prepared not expanded stents are weighed and horizontally hung onto a thin metal bar (d = 0.2 mm) which is stuck on the rotation axis of the rotation and feed equipment and rotates with 28 r/min. The stents are fixed in such way, that the interior of the stents does not touch the bar. At a feeding amplitude of 2.2 cm and a feeding velocity of 4 cm/s and a distance of 6 cm between stent and spray nozzle, the stent is sprayed with the pure active agent spray solution. After the drying (about 15 minutes) at room temperature and leaving in the fume hood over night the stent is weighed again.

### Example 2. Dip coating of bare metal stent with pure active agent

Dip method: The according to Example 1 pre-prepared not expanded stents are dipped into the according to Example 1 prepared pure active agent solution. After a couple of minutes the stent is taken out of the solution with tweezers and moved within the fume hood until the solvent evaporates. Then the stent is dipped in for a second time. After air drying the stent is freeze dried for about 10 min. The stents are weighed before the dipping procedure and after freeze drying.

### Example 3. Covalent hemocompatible coating of metal stents

Not expanded stents of medicinal stainless steel LVM 316 are degreased in the ultrasonic bath for 15 minutes with acetone and ethanol and dried at 100°C in the drying closet. Then they are dipped for 5 minutes into a 2% solution of 3_aminopropyltriethoxysilane in a mixture of ethanol/water (50/50 : (v/v)) and then dried for 5 minutes at 100°C. Afterwards the stents are washed with demineralised water over night.

3 mg desulphated and reacetylated heparin is dissolved at 4°C in 30 ml 0.1 M MES-buffer (2-(N-morpholino)ethanesulphonic acid) pH 4.75 and mixed with 30 mg N-cyclohexyl-N'-(2-morpholinoethyl)carbodiimide-methyl-p-toluenesulphonate. In this solution 10 stents are stirred for 15 hours at 4°C. Then they are rinsed with water, 4 M NaCl solution and water in each case for 2 hours.

### Example 4. Spray coating of a bare metal stent with biodegradable matrix solution

Preparation of the spray solution: 176 mg polylactide is balanced and reconstituted with chloroform to 20 g.
The stents prepared and are sprayed, as described in Example 1, in each case with 3 ml of the spraying solution, weighed before and after the spraying and the yielding layer thickness is determined by measuring under the microscope 100-times magnified.
If a gemcitabine compound such as gemcitabine, gemcitabine hydrochloride or any other gemcitabine salt or gemcitabine derivative optionally together with one or two further active agents should be incorportated in the biodegradable layer, a suitable amount of the gemcitabine compound and the optionally further active agents are dissolved or dispersed in the spray solution.

### Example 5. Spray coating of bare stents with active agent loaded biodegradable matrix solution

Preparation of the spray solution: 145.2 mg polylactide and 48.4 mg gemcitabine is reconstituted to 22 g with chloroform. The form of gemcitabine used is preferably gemcitabine free base, gemcitabine hydrochloride or the gemcitabine derivative as disclosed in Formula (IV) wherein R⁵ is eicosanoic acid or DHA.

The stents are prepared and are sprayed, as described in Example 1, in each case with 3 ml of the spraying solution, weighed before and after the spraying and the yielding layer thickness is determined by measuring under the microscope 100-times magnified.

### Example 6. Preparation of stents having a hemocompatible and/or biodegradable base coating and an active agent loaded matrix coating.

Coated stents as prepared in Examples 3 or 4 are subsequently coated with an active agent loaded biodegradable matrix solution as described in Example 5. After the first base base coating is applied a time period of at least 6 hours passes until the active agent loaded matrix coating is applied. The stents are weighed before and after the application of each coating.

### Example 7. Preparation of stents having a hemocompatible and/or biodegradable base coating, an active agent loaded matrix coating and a biodegradable outer coat.

Coated stents as prepared in Examples 3 or 4 are subsequently coated with an active agent loaded biodegradable matrix solution as described in Example 5. The resulting stents are then further coated with a biodegradable outer coat as described in Example 4. After the first base base coating is applied a time period of at least 6 hours passes until the active agent loaded matrix coating is applied and after the active agent loaded matrix coating is applied a time period of at least 6 hours passes until the biodegradable outer coat is applied. The stents are weighed before and after the application of each coating.

### Example 8. Coating of stents with polyethersulfone

Alternatively the base coat used on the stents in Examples 6 and 7 can be a polyethersulfone coating applied by the spray method or the dip method as described in Examples 1 and 2 using a polyethersulfone solution.
Polyethersulfone solution: 176 mg of PS (polyethersulfone, Udel®, available from Solvay) are weighed in and filled up to 20 g with chloroform.
→ 0.88 % PS

### Example 9. Coating of stents with polyethersulfone/gemcitabine matrix coating

Alternatively the active agent loaded matrix coating used on the stents in Examples 6 and 7 can be a polyethersulfone matrix coating applied by the spray method or the dip method as described in Examples 1 and 2 using a polyethersulfone/gemcitabine matrix solution.
Polyethersulfone/gemcitabine solution: 13.2 mg of PS and 4.4 mg of gemcitabine are weighed in and filled up to 2 g with chloroform.
→ 0.66 % of PS, 0.22 % of gemcitabine

### Example 10. Coating of stents with polyethersulfone/PVP outer coat

Alternatively the outer coat used on the stents in Example 7 can be a polyethersulfone/PVP top coating applied by the spray method or the dip method as described in Examples 1 and 2 using a polyethersulfone/PVP solution A or B.
Polyethersulfone solution A: 25.2 mg of PS and 1.2 mg of PVP are weighed in and filled up to 3 g with chloroform.
→ 0.84 % of PS, 0.04 % of PVP
Polyethersulfone solution B: 24 mg of PS and 2.4 mg of PVP are weighed in and filled up to 3 g with chloroform.
→ 0.80 % of PS, 0.08 % of PVP

### Example 11. Use of further active ingredients in the gemcitabine containing active agent loaded matrix coating

The active agent loaded matrix coating of Example 5 or Example 9 can also contain a further active ingredient preferably selected from paclitaxel, rapamycin, cyclosporin A, tacrolimus, mycophenolate mofetil, or daclizumab.

### Example 12. Use of further active ingredients in an active agent loaded matrix coating

A further active agent loaded matrix coating containing an active ingredient preferably selected from paclitaxel, rapamycin, cyclosporin A, tacrolimus, mycophenolate mofetil, or daclizumab can be applied above or below the gemcitabine containing active agent loaded matrix coating of Example 5 or Example 9.

### Example 13. In vivo examination of stents with and without gemcitabine

Into the coronary arteries of 13 domestic pigs of different sex with a weight of 20-25 kg are implanted stents prepared according to Examples 6 or 7 with and without gemicitabine. After four weeks, the stents are removed and analyzed for inflammation reactions (peri-strut) and formation of neointima. Thickness of intima, stenosis and grade of injury are histomorphometrically evaluated.

### Example 14. Coating of a catheter balloon with gemcitabine

### a) Coating an expanded balloon catheter - Method A

A standard balloon catheter, for example Joker Lite made by BMT, Oberpfaffenhofen/Munich, Germany, balloon dimensions 2.5 mm by 20 mm, is inflated to the maximum and immersed full length for 1 minute in ethyl acetate, 18.8 mg gemcitabine per ml, +1 % pharmaceutical olive oil, dried.

### b) Coating an expanded balloon catheter - Method B

Standard balloon catheters, for example Joker Lite made by BMT, Oberpfaffenhofen/Munich, Germany, balloon dimensions 2.5 mm by 20 mm, are inflated to the maximum and immersed full length in a solution of 350 mg of gemcitabine dissolved in 9.0 ml of acetone for 1 minute and removed. The solvent is dried for 12 hours at room temperature. Then the balloon is deflated and folded in the common way using a PTFE-coated tool. Optionally, one can crimp a stent of suitable dimensions onto the balloon.

### c) Coating a folded balloon catheter - Method A

Folded balloon catheters, for example made by BMT, Oberpfaffenhofen/Munich, Germany, product name Joker Lite, balloon dimensions 2.5 mm by 20 mm, are immersed full length in folded condition for 1 minute in ethyl acetate, 18.8 mg gemcitabine per ml, +1% pharmaceutical olive oil, and dried.

### c) Coating a folded balloon catheter - Method B

a) Folded balloon catheters, for example Joker Lite made by BMT, Oberpfaffenhofen/Munich, Germany, balloon dimensions 2.5 mm by 20 mm, are immersed full length in folded condition for 1 minute in ethyl acetate, 16.6 mg gemcitabine per ml, and dried for 4 hours. To increase gemcitabine content immerse an additional two to four times for 5 seconds with 1 hour drying time after each immersion process in solution A (=3.33 ml ethyl acetate +100.0 mg of gemcitabine).

### Example 15. Coating of a catheter balloon with gemcitabine and a contrast agent

Folded balloon catheters, for example Allegro made by BMT, balloon dimensions 2.5 by 20 mm, are immersed in a mixture of 0.15 ml ethanol, 4.5 [mu]l of Ultravist 300 (an X-ray contrast agent made by Schering AG, Berlin, Germany), 1.35 ml of acetone, 0.8 mg of Sudan red and 30.0 mg of gemcitabine. The folded balloon sections of the catheters are immersed 5 times, the first time for one minute, then dried for 3 hours, then immersed 4 times at 1 hour intervals for 5 seconds each. Subsequently, a stent is crimped on and the catheter is sterilized in the common way using ethylene oxide.

### Example 16. Bioresorbable metal stent with biodegradable coating containing DHA gemcitabine

A bioresorbable metal stent is made from an alloy in which the component A consists of zinc and the component B of calcium according to the procedures disclosed in EP 1 419 793 B1, EP 0 966 979 A2, DE 102 07 161 A1, DE 102 53 634 A1 and DE 102 37 572 A1. The weight ratio that zinc bears to calcium amounts to at least 21:1. This Zn-Ca alloy is an alloy in which the corrosion products of both components are soluble salts, which is of special advantage in the case of the present field of application. Calcium hydroxide possesses so high a solubility that the solubility product is not transgressed during slow corrosion over several weeks or months. The coronary hydroxide is transported in dissolved form by the blood and subjected to metabolism.

Solution of DHA-gemcitabine (a gemcitabine derivative according to general formual (IV), wherein R⁵CO represents the DAH (4,7,10,13,16,19-docosahexaenoic) fatty acid residue), prepared in a solvent miscible with polymer carrier solution, is mixed with solution of polymer at final concentration range 0.001 weight % to 30 weight % of drug. Polymers used are biocompatible (i.e., not elicit any negative tissue reaction or promote mural thrombus formation) and degradable, such as lactone-based polyesters or copolyesters, e.g., polylactide, polycaprolactonglycolide,polyorthoesters, polyanhydrides; poly-aminoacids; polysaccharides; polyphosphazenes; poly(ether-ester) copolymers, e.g., PEO-PLLA, or blends thereof. In the present case a biodegradable polyester and a biodegradable polysaccharide was used in two different embodiments.

Polymer/DHA-gemcitabine mixture is applied to the surfaces of the stent by either dip-coating, or spray coating, or brush coating or dip/spin coating or combinations thereof, and the solvent allowed to evaporate to leave a film with entrapped DHA-gemcitabine.

### Example 17. Bioresorbable chitosan stent with gemcitabine

Chitosan powder is dissolved in acetic acid at about pH 4 by dispersing 3 grams powder in 50 ml of water containing 0.5 wt % acetic acid. Chitosan (MW: about 70,000) was purchased from Fluka Chemical Co. (Buchs, Switzerland). The chitosan polymer solution was prepared by mechanical stirring at about 600 rpm for about 3 hours until all powder is dissolved. Subsequently, the chitosan solution was adjusted to approximately pH 5.5 (right before it becomes gelled) with NaOH. If a gemcitabine compound should be incorporated the chitosan stent, add in at least one gemcitabine compound such as gemcitabine tosylate, or gemcitabine mesylate into the chitosan solution. While loading the gemcitabine containing chitosan onto a mold, the environment was adjusted to pH 7 with NaOH to solidify the chitosan to make a stent. In one example, the mold is a helically bendable hollow mold (such as the one made of silicone or polyurethane-silicone copolymer). During the solidification stage, the mold is promptly bent helically or spirally. After the chitosan is fully solidified, the mold was removed to obtain a shaped chitosan pre-product. In another example, a cylindrical mold is used to make a cast chitosan film onto the inner surface of the cylindrical mold. During the solidification stage, the mold was rotated at a desired speed, say, several hundred to several thousand rpm. The cylindrical film, after solidified, was thereafter cut by a spiral knife to make a spiral chitosan pre-product. In a third example, the solidifiable solution is made into films, whereas the films was cut into strips of about 2 mm wide. These strips were then wound onto a mandrill and means for forming the helical pre-product was applied, wherein the means may comprise heat set or other change in the environment conditions.

If the at least one gemcitabine compound should not be incorporated into the stent struts itself, or in addition to the incorporated at least one gemcitabine compound a coating can be applied on the surface of the biodegradable stent. According to the coating procedures of the afore-mentioned examples, the bioresorbable stent can be at least partially covered with a biostable or biodegradable layer optionally containing active agents.

## Claims

1. Use of at least one gemcitabine compound for the manufacture of a pharmaceutical composition for prophylaxis and/or treatment of cardiovascular diseases and disorders and cardiovascular injuries.

2. Use of at least one gemcitabine compound as coating material for an expandable medical device.

3. Use according to claim 1 or 2, wherein the at least one gemcitabine compound is used in combination with at least one other anti-proliferative, anti-migrative, anti-inflammatory, anti-phlogistic, anti-restenosis, anti-angiogenic and/or anti-thrombotic active agent.

4. Use according to claim 3, wherein the at least one other anti-proliferative, anti-migrative, anti-inflammatory, anti-phlogistic, anti-restenosis, anti-angiogenic and/or anti-thrombotic active agent is selected from the group consisting of: sirolimus (rapamycin), everolimus, somatostatin, tacrolimus, roxithromycin, dunaimycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoposide, teniposide, nimustine, carmustine, lomustine, cyclophosphamide, C-type natriuretic peptide (CNP), 4-hydroxycyclophosphamide, estramustine, melphalan, ifosfamide, trofosfamide, chlorambucil, bendamustine, dacarbazine, busulfan, procarbazine, treosulfan, temozolomide, thiotepa, daunorubicin, doxorubicin, aclarubicin, epirubicin, mitoxantrone, idarubicin, bleomycin, mitomycin, dactinomycin, methotrexate, fludarabine, fludarabine-5'-dihydrogenphosphate, cladribine, mercaptopurine, thioguanine, cytarabine, fluorouracil, capecitabine, docetaxel, carboplatin, cisplatin, cryptophycine, anginex, oxaliplatin, amsacrine, irinotecan, topotecan, hydroxycarbamide, miltefosine, pentostatin, aldesleukin, tretinoin, asparaginase, pegaspargase, anastrozole, exemestane, letrozole, formestane, aminoglutethimide, adriamycin, azithromycin, spiramycin, cepharantin, smc proliferation inhibitor-2w, epothilone A and B, mitoxantrone, azathioprine, mycophenolatmofetil, c-myc-antisense, b-myc-antisense, betulinic acid, camptothecin, lapachol, β-lapachone, podophyllotoxin, betulin, podophyllic acid 2-ethylhydrazide, molgramostim (rhuGM-CSF), peginterferon α-2b, lenograstim (r-HuG-CSF), filgrastim, macrogol, anginex, Na-Uretic peptides, dacarbazine, basiliximab, daclizumab, selectin (cytokine antagonist), chryptophycines, CETP inhibitor, cadherines, cytokinin inhibitors, COX-2 inhibitor, AE-941 (Neovastat®) NFkB, angiopeptin, ciprofloxacin, camptothecin, fluroblastin, monoclonal antibodies, which inhibit the muscle cell proliferation, bFGF antagonists, probucol, prostaglandins, Ac-YVAD-CMK, 1,11-dimethoxycanthin-6-one, 1-hydroxy-11-methoxycanthin-6-one, scopoletin, colchicine, NO donors such as pentaerythritol tetranitrate and syndnoeimines, S-nitrosoderivatives, tamoxifen, staurosporine, β-estradiol, α-estradiol, estriol, estrone, ethinylestradiol, fosfestrol, medroxyprogesterone, estradiol cypionates, estradiol benzoates, tranilast, kamebakaurin and other terpenoids, which are applied in the therapy of cancer, verapamil, tyrosine kinase inhibitors (tyrphostines), cyclosporine A, paclitaxel and its derivatives such as 6-α-hydroxy-paclitaxel, baccatin, taxotere and others, synthetically produced as well as macrocyclic oligomers obtained from native sources of carbon suboxide (MCS) and its derivatives, mofebutazone, acemetacin, diclofenac, lonazolac, dapsone, o-carbamoylphenoxyacetic acid, lidocaine, ketoprofen, mefenamic acid, piroxicam, meloxicam, chloroquine phosphate, penicillamine, hydroxychloroquine, auranofin, sodium aurothiomalate, oxaceprol, celecoxib, β-sitosterin, ademetionine, myrtecaine, polidocanol, nonivamide, levomenthol, benzocaine, aescin, ellipticine, D-24851 (Calbiochem), colcemid, cytochalasin A-E, indanocine, nocodazole, S 100 protein, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor-1, plasminogen activator inhibitor-2, antisense oligonucleotides, VEGF inhibitors, IGF-1, active agents from the group of antibiotics such as cefadroxil, cefazolin, cefaclor, cefotaxim, tobramycin, gentamycin, penicillins such as dicloxacillin, oxacillin, sulfonamides, metronidazol, antithrombotics such as argatroban, aspirin, abciximab, synthetic antithrombin, bivalirudin, coumadin, enoxaparin, desulphated and N-reacetylated heparin (hemoparin®), tissue plasminogen activator, GpIIb/IIIa platelet membrane receptor, factor Xₐ inhibitor antibody, heparin, hirudin, r-hirudin, PPACK, protamin, prourokinase, streptokinase, warfarin, urokinase, vasodilators such as dipyramidole, triazolopyrimidine (trapidil®), nitroprussides, PDGF antagonists such as triazolopyrimidine and seramin, ACE inhibitors such as captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators such as p65 NF-kB or Bcl-xL antisense oligonucleotides, halofuginone, nifedipine, tocopherol, tranilast, molsidomine, tea polyphenols, epicatechin gallate, epigallocatechin gallate, Boswellic acids and its derivatives, leflunomide, anakinra, etanercept, sulfasalazine, etoposide, dicloxacillin, tetracycline, triamcinolone, mutamycin, procainamid, retinoic acid, quinidine, disopyrimide, flecainide, propafenone, sotalol, amidorone, natural and synthetically produced steroids such as bryophyllin A, inotodiol, maquiroside A, ghalakinoside, mansonine, strebloside, hydrocortisone, betamethasone, dexamethasone, non-steroidal substances (NSAIDS) such as fenoprofen, ibuprofen, indomethacin, naproxen, phenylbutazone and other antiviral agents such as acyclovir, ganciclovir and zidovudine, antimycotics such as clotrimazole, flucytosine, griseofulvin, ketoconazole, miconazole, nystatin, terbinafine, antiprozoal agents such as chloroquine, mefloquine, quinine, moreover natural terpenoids such as hippocaesculin, barringtogenol-C21-angelate, 14-dehydroagrostistachin, agroskerin, agrostistachin, 17-hydroxyagrostistachin, ovatodiolids, 4,7-oxycycloanisomelic acid, baccharinoids B1, B2, B3 and B7, tubeimoside, bruceanol A, B and C, bruceantinoside C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18- dehydro-6-α-senecioyloxychaparrin, taxamairin A and B, regenilol, triptolide, furthermore cymarin, apocymarin, aristolochic acid, anopterin, hydroxyanopterin, anemonin, protoanemonin, berberine, cheliburin chloride, cictoxin, sinococuline, bombrestatin A and B, cudraisoflavone A, curcumin, dihydronitidine, nitidine chloride, 12-β-hydroxypregnadiene-4,16-diene-3,20-dione, bilobol, ginkgol, ginkgolic acid, helenalin, indicine, indicine-N-oxide, lasiocarpine, inotodiol, glycoside 1a, podophyllotoxin, justicidin A and B, larreatin, malloterin, mallotochromanol, isobutyrylmallotochromanol, maquiroside A, marchantin A, maytansine, lycoridicin, margetine, pancratistatin, liriodenine, bisparthenolidine, oxoushinsunine, aristolactam-All, bisparthenolidine, periplocoside A, bisparthenolidine, periplocoside A, ghalakinoside, ursolic acid, deoxypsorospermin, psychorubin, ricin A, sanguinarine, manwu wheat acid, methylsorbifolin, sphatheliachromen, stizophyllin, mansonine, strebloside, akagerine, dihydrousambarensine, hydroxyusambarine, strychnopentamine, strychnophylline, usambarine, usambarensine, berberine, liriodenine, oxoushinsunine, daphnoretin, lariciresinol, methoxylariciresinol, syringaresinol, umbelliferon, afromoson, acetylvismione B, desacetylvismione A, vismione A and B and mixtures of these agents.

5. Use according to claim 2, wherein the at least one gemcitabine compound is used together with polymeric substances, contrast agents and/or pharmaceutically acceptable carriers.

6. Use according to any previous claim, wherein the cardiovascular diseases and disorders are selected from the group consisting of adult congenital heart disease, aneurysm, stable angina, unstable angina, angina pectoris, angioneurotic edema, aortic valve stenosis, aortic aneurysm, arrhythmia, arrhythmogenic right ventricular dysplasia, arteriosclerosis, atherosclerosis, arteriovenous malformations, atrial fibrillation, Behcet syndrome, bradycardia, cardiac tamponade, cardiomegaly, congestive cardiomyopathy, hypertrophic cardiomyopathy, restrictive cardiomyopathy, cardiovascular disease prevention, carotid stenosis, cerebral hemorrhage, Churg-Strauss syndrome, diabetes, Ebstein's Anomaly, Eisenmenger complex, cholesterol embolism, bacterial endocarditis, fibromuscular dysplasia, congenital heart defects, heart diseases, congestive heart failure, heart valve diseases, heart attack, epidural hematoma, hematoma, subdural, Hippel-Lindau disease, hyperemia, hyperproliferative vascular disease, intimal hyperplasia, hypertension, pulmonary hypertension, hypertrophic growth, left ventricular hypertrophy, right ventricular hypertrophy, hypoplastic left heart syndrome, hypotension, intermittent claudication, ischemic heart disease, Klippel-Trenaunay-Weber syndrome, lateral medullary syndrome, long QT syndrome mitral valve prolapse, moyamoya disease, mucocutaneous lymph node syndrome, myocardial infarction, myocardial ischemia, myocarditis, pericarditis, peripheral vascular diseases, phlebitis, polyarteritis nodosa, pulmonary atresia, Raynaud disease, restenosis, in-stent restenosis, Sneddon syndrome, stenosis, superior vena cava syndrome, syndrome X, tachycardia, Takayasu's arteritis, hereditary hemorrhagic telangiectasia, telangiectasis, temporal arteritis, tetralogy of fallot, thromboangiitis obliterans, thrombosis, thromboembolism, tricuspid atresia, varicose veins, vascular diseases, vasculitis, vasospasm, ventricular fibrillation, Williams syndrome, peripheral vascular disease, varicose veins and leg ulcers, deep vein thrombosis, Wolff-Parkinson-White syndrome and the cardiovascular injuries are selected from the group consisting of mechanically mediated vascular injury, vascular catheterization, vascular scraping, percutaneous transluminal coronary angioplasty, vascular surgery and vascular laser treatment.

7. Use according to any previous claim, wherein the expandable medical device is selected from the group consisting of grafts, vascular grafts, stents, vascular stent, urological stent, biliary stent, bronchial stent, urinary stent, gastrointestinal stent, peripheral stent, catheter balloon, PTCA balloon, angiography ballon, and dilatation balloon.

8. Use according to any previous claim, wherein the gemcitabine compound is gemcitabine.

9. Expandable medical device, wherein at least one gemcitabine compound is present on the surface of said expandable medical device.

10. Expandable medical device according to claim 9, wherein the at least one gemcitabine compound is used as pure chemical substance on the surface of the expandable medical device or in cavities in the surface of the expandable medical device or incorporated in a polymeric matrix coated on the surface of the expandable medical device or mixed with at least one pharmaceutically acceptable carrier coated on the surface of the expandable medical device or deposited into folds or crinkles or wings of the expandable medical device.

11. Expandable medical device according to claim 10, wherein the polymeric matrix comprising the at least one gemcitabine compound consists of polymers or oligomers selected from the group consisting of polyvalerolactones, poly-ε-decalactones, polylactonic acid, polyglycolic acid, polylactides, polyglycolides, copolymers of the polylactides and polyglycolides, poly-ε-caprolactone, polyhydroxybutanoic acid, polyhydroxybutyrates, polyhydroxyvalerates, polyhydroxybutyrate-co-valerates, poly(1,4-dioxane-2,3-diones), poly(1,3-dioxane-2-one), poly-para-dioxanones, polyanhydrides, polymaleic anhydrides, polyhydroxymethacrylates, fibrin, polycyanoacrylates, polycaprolactonedimethylacrylates, poly-β-maleic acid, polycaprolactonebutyl-acrylates, multiblock polymers from oligocaprolactonedioles and oligodioxanonedioles, polyetherester multiblock polymers from PEG and poly(butyleneterephtalates), polypivotolactones, polyglycolic acid trimethyl-carbonates, polycaprolactone-glycolides, poly(γ-ethylglutamate), poly(DTH-iminocarbonate), poly(DTE-co-DT-carbonate), poly(bisphenol-A-iminocarbonate), polyorthoesters, polyglycolic acid trimethyl-carbonates, polytrimethylcarbonates, polyiminocarbonates, poly(N-vinyl)-pyrrolidone, polyvinylalcohols, polyesteramides, glycolated polyesters, polyphosphoesters, polyphosphazenes, poly[p-carboxyphenoxy)propane], polyhydroxypentane acid, polyanhydrides, polyethyleneoxide-propyleneoxide, soft polyurethanes, polyurethanes with amino acid residues in the backbone, polyetheresters as polyethyleneoxide, polyalkeneoxalates, polyorthoesters, copolymers of polyorthoesters, lipids, carrageenans, fibrinogen, starch, collagen, protein based polymers, polyamino acids, synthetic polyamino acids, zein, modified zein, polyhydroxyalkanoates, pectic acid, actinic acid, casein, carboxymethylsulphate, albumin, moreover hyaluronic acid, chitosane, derivatives of chitosane, heparansulphates, derivatives of heparansulphates, heparin, chondroitinsulphate, dextran, β-cyclodextrins, copolymers with PEG and polypropyleneglycol, gum arabicum, guar, gelatine, collagen, collagen-N-hydroxysuccinimide, lipids, phospholipids, polyacrylic acid, polyacrylates, polymethylmethacrylate, polybutylmethacrylate, polyacrylamide, polyacrylonitriles, polyamides, polyetheramides, polyethylenamine, polyimides, polycarbonates, polycarbourethanes, polyvinylketones, polyvinylhalogenides, polyvinylidenhalogenides, polyvinylethers, polyisobutylenes, polyvinylaromates, polyvinylesters, polyvinylpyrollidones, polyoxymethylenes, polytetramethyleneoxide, polyethylene, polypropylene, polytetrafluoroethylene, polyurethanes, polyetherurethanes, silicone-polyetherurethanes, silicone-polyurethanes, silicone-polycarbonate-urethanes, polyolefine elastomeres, polyisobutylenes, EPDM gums, fluorosilicones, carboxymethylchitosans, polyaryletheretherketones, polyetheretherketones, polyethylenterephthalate, polyvalerates, carboxymethylcellulose, cellulose, rayon, rayontriacetates, cellulosenitrates, celluloseacetates, hydroxyethylcellulose, cellulosebutyrates, celluloseacetatebutyrates, ethylvinylacetate copolymers, polysulphones, epoxy resins, ABS resins, EPDM gums, silicones as polysiloxanes, polydimethylsiloxanes, polyvinylhalogenes, copolymers containing polyvinylhalogenes, celluloseethers, cellulosetriacetates, chitosans and copolymers and/or mixtures of these substances.

12. Expandable medical device according to claim 10, wherein pharmaceutically acceptable carrier is selected from the group consisting of contrast agents, lactose, starch, sucrose, magnesium stearate, dicalcium phosphate, calcium sulfate, talc, mannitol, magnesium aluminum silicate, natural sugars, acacia, polyethylene glycol, waxes, guar gum, boric acid, sodium benzoate, sodium acetate, sodium chloride, acacia, gelatin, tragacanth, alginic acid, sodium alginate, ammonium calcium alginate, cellulose, methylcellulose, sodium carboxymethylcellulose, hydroxypropylmethylcellulose, polyvinylpyrrolidone.

13. Expandable medical device according to claim 12, wherein the contrast agents are selected from the group consisting of amidotrizoic acid, iotrolan, iodoxamic acid, ioglycamic acid, iopromide, Ultravist^{®}, iopamidol, iohexol, ioxilan, iomeprol, ioversol, iobitridol, iothalamate, meglumine, Telebrix^{®}, Omnipaque^{®}, gadopentenoic acid, gadodiamide, meglumin-gadoterate and gadoteridol.

14. Expandable medical device according to any one of claims 9-13, wherein a hemocompatible layer is present on the surface of the expandable medical device and below the gemcitabine compound containing coating.

15. Expandable medical device according to any one of claims 9 - 14, wherein a barrier layer is present on top of the gemcitabine compound containing coating.

16. Expandable medical device according to any one of claims 9-15, wherein the at least one gemcitabine compound is used in combination with at least one other anti-proliferative, anti-migrative, anti-inflammatory, anti-phlogistic, anti-restenosis, anti-angiogenic and/or anti-thrombotic active agent.

17. Expandable medical device according to claim 16, wherein the at least one other anti-proliferative, anti-migrative, anti-inflammatory, anti-phlogistic, anti-restenosis, anti-angiogenic and/or anti-thrombotic active agent is selected from the group consisting of: sirolimus (rapamycin), everolimus, somatostatin, tacrolimus, roxithromycin, dunaimycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoposide, teniposide, nimustine, carmustine, lomustine, cyclophosphamide, C-type natriuretic peptide (CNP), 4-hydroxycyclophosphamide, estramustine, melphalan, ifosfamide, trofosfamide, chlorambucil, bendamustine, dacarbazine, busulfan, procarbazine, treosulfan, temozolomide, thiotepa, daunorubicin, doxorubicin, aclarubicin, epirubicin, mitoxantrone, idarubicin, bleomycin, mitomycin, dactinomycin, methotrexate, fludarabine, fludarabine-5'-dihydrogenphosphate, cladribine, mercaptopurine, thioguanine, cytarabine, fluorouracil, capecitabine, docetaxel, carboplatin, cisplatin, cryptophycine, anginex, oxaliplatin, amsacrine, irinotecan, topotecan, hydroxycarbamide, miltefosine, pentostatin, aldesleukin, tretinoin, asparaginase, pegaspargase, anastrozole, exemestane, letrozole, formestane, aminoglutethimide, adriamycin, azithromycin, spiramycin, cepharantin, smc proliferation inhibitor-2w, epothilone A and B, mitoxantrone, azathioprine, mycophenolatmofetil, c-myc-antisense, b-myc-antisense, betulinic acid, camptothecin, lapachol, β-lapachone, podophyllotoxin, betulin, podophyllic acid 2-ethylhydrazide, molgramostim (rhuGM-CSF), peginterferon α-2b, lenograstim (r-HuG-CSF), filgrastim, macrogol, anginex, Na-Uretic peptides, dacarbazine, basiliximab, daclizumab, selectin (cytokine antagonist), chryptophycines, CETP inhibitor, cadherines, cytokinin inhibitors, COX-2 inhibitor, AE-941 (Neovastat^{®}) NFkB, angiopeptin, ciprofloxacin, camptothecin, fluroblastin, monoclonal antibodies, which inhibit the muscle cell proliferation, bFGF antagonists, probucol, prostaglandins, Ac-YVAD-CMK, 1,11-dimethoxycanthin-6-one, 1-hydroxy-11-methoxycanthin-6-one, scopoletin, colchicine, NO donors such as pentaerythritol tetranitrate and syndnoeimines, S-nitrosoderivatives, tamoxifen, staurosporine, β-estradiol, α-estradiol, estriol, estrone, ethinylestradiol, fosfestrol, medroxyprogesterone, estradiol cypionates, estradiol benzoates, tranilast, kamebakaurin and other terpenoids, which are applied in the therapy of cancer, verapamil, tyrosine kinase inhibitors (tyrphostines), cyclosporine A, paclitaxel and its derivatives such as 6-α-hydroxy-paclitaxel, baccatin, taxotere and others, synthetically produced as well as macrocyclic oligomers obtained from native sources of carbon suboxide (MCS) and its derivatives, mofebutazone, acemetacin, diclofenac, lonazolac, dapsone, o-carbamoylphenoxyacetic acid, lidocaine, ketoprofen, mefenamic acid, piroxicam, meloxicam, chloroquine phosphate, penicillamine, hydroxychloroquine, auranofin, sodium aurothiomalate, oxaceprol, celecoxib, β-sitosterin, ademetionine, myrtecaine, polidocanol, nonivamide, levomenthol, benzocaine, aescin, ellipticine, D-24851 (Calbiochem), colcemid, cytochalasin A-E, indanocine, nocodazole, S 100 protein, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor-1, plasminogen activator inhibitor-2, antisense oligonucleotides, VEGF inhibitors, IGF-1, active agents from the group of antibiotics such as cefadroxil, cefazolin, cefaclor, cefotaxim, tobramycin, gentamycin, penicillins such as dicloxacillin, oxacillin, sulfonamides, metronidazol, antithrombotics such as argatroban, aspirin, abciximab, synthetic antithrombin, bivalirudin, coumadin, enoxaparin, desulphated and N-reacetylated heparin (hemoparin®), tissue plasminogen activator, GpIIb/IIIa platelet membrane receptor, factor Xₐ inhibitor antibody, heparin, hirudin, r-hirudin, PPACK, protamin, prourokinase, streptokinase, warfarin, urokinase, vasodilators such as dipyramidole, triazolopyrimidine (trapidil^{®}), nitroprussides, PDGF antagonists such as triazolopyrimidine and seramin, ACE inhibitors such as captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators such as p65 NF-kB or Bcl-xL antisense oligonucleotides, halofuginone, nifedipine, tocopherol, tranilast, molsidomine, tea polyphenols, epicatechin gallate, epigallocatechin gallate, Boswellic acids and its derivatives, leflunomide, anakinra, etanercept, sulfasalazine, etoposide, dicloxacillin, tetracycline, triamcinolone, mutamycin, procainamid, retinoic acid, quinidine, disopyrimide, flecainide, propafenone, sotalol, amidorone, natural and synthetically produced steroids such as bryophyllin A, inotodiol, maquiroside A, ghalakinoside, mansonine, strebloside, hydrocortisone, betamethasone, dexamethasone, non-steroidal substances (NSAIDS) such as fenoprofen, ibuprofen, indomethacin, naproxen, phenylbutazone and other antiviral agents such as acyclovir, ganciclovir and zidovudine, antimycotics such as clotrimazole, flucytosine, griseofulvin, ketoconazole, miconazole, nystatin, terbinafine, antiprozoal agents such as chloroquine, mefloquine, quinine, moreover natural terpenoids such as hippocaesculin, barringtogenol-C21-angelate, 14-dehydroagrostistachin, agroskerin, agrostistachin, 17-hydroxyagrostistachin, ovatodiolids, 4,7-oxycycloanisomelic acid, baccharinoids B1, B2, B3 and B7, tubeimoside, bruceanol A, B and C, bruceantinoside C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-α-senecioyloxychaparrin, taxamairin A and B, regenilol, triptolide, furthermore cymarin, apocymarin, aristolochic acid, anopterin, hydroxyanopterin, anemonin, protoanemonin, berberine, cheliburin chloride, cictoxin, sinococuline, bombrestatin A and B, cudraisoflavone A, curcumin, dihydronitidine, nitidine chloride, 12-β-hydroxypregnadiene-4,16-diene-3,20-dione, bilobol, ginkgol, ginkgolic acid, helenalin, indicine, indicine-N-oxide, lasiocarpine, inotodiol, glycoside 1 a, podophyllotoxin, justicidin A and B, larreatin, malloterin, mallotochromanol, isobutyrylmallotochromanol, maquiroside A, marchantin A, maytansine, lycoridicin, margetine, pancratistatin, liriodenine, bisparthenolidine, oxoushinsunine, aristolactam-All, bisparthenolidine, periplocoside A, bisparthenolidine, periplocoside A, ghalakinoside, ursolic acid, deoxypsorospermin, psychorubin, ricin A, sanguinarine, manwu wheat acid, methylsorbifolin, sphatheliachromen, stizophyllin, mansonine, strebloside, akagerine, dihydrousambarensine, hydroxyusambarine, strychnopentamine, strychnophylline, usambarine, usambarensine, berberine, liriodenine, oxoushinsunine, daphnoretin, lariciresinol, methoxylariciresinol, syringaresinol, umbelliferon, afromoson, acetylvismione B, desacetylvismione A, vismione A and B and mixtures of these agents.

18. Expandable medical device according to any one of claims 9 - 17, wherein the at least one gemcitabine compound is used in an amount of 1 µg / mm² to 20 µg / mm² surface of the expandable medical device.

19. Expandable medical device according to any one of claims 9 - 18, wherein the at least one other anti-proliferative, anti-migrative, anti-inflammatory, anti-phlogistic, anti-restenosis, anti-angiogenic and/or anti-thrombotic active agent is contained in a pharmaceutically active concentration of 0.001 - 20 mg per cm² of the surface of the expandable medical device.

20. Expandable medical device according to any one of claims 9 - 19, wherein the expandable medical device is selected from the group consisting of grafts, vascular grafts, stents, vascular stent, urological stent, biliary stent, bronchial stent, urinary stent, gastrointestinal stent, peripheral stent, catheter balloon, PTCA balloon, angiography ballon, and dilatation balloon.

21. Expandable medical device according to claim 20, wherein the expandable medical device is a stent coated with a polymeric carrier containing the at least one gemcitabine compound.

22. Expandable medical device according to claim 20, wherein the expandable medical device is a dilatation balloon coated with a mixture containing gemcitabine and at least one contrast agent.

23. Expandable medical device according to any one of claims 9 - 22, wherein the at least one gemcitabine compound is gemcitabine.

24. Coating method for a expandable medical device comprising the following steps:
a) providing an expandable medical device, and
b) depositing the at least one gemcitabine compound as pure substance or in combination with at least one pharmaceutically acceptable carrier or with a polymeric carrier on the surface of the expandable medical device or in cavities in the surface of the expandable medical device or into folds or crinkles or wings of the expandable medical device.

25. Coating method according to claim 24, comprising the steps:
a) providing an expandable medical device, and
a') applying a layer of a hemocompatible material on the surface of the expandable medical device, and
b) depositing the at least one gemcitabine compound as pure substance or in combination with at least one pharmaceutically acceptable carrier or with a polymeric carrier on the hemocompatible surface of the expandable medical device.

26. Coating method according to claim 24 or 25, comprising the further step
c) depositing at least one further layer of at least one biodegradable polymer on the at least one gemcitabine compound or on the at least one gemcitabine compound containing layer.

27. Expandable medical device obtainable according to a coating method as defined in claim 24, 25 and 26.
